# EUROPEAN PATENT SPECIFICATION

(11) **EP 2 755 599 B1**
(45) Date of publication and mention of the grant of the patent: **01.04.2020**
(21) Application number: 12831717.9
(22) Date of filing: 12.09.2012
(51) Int. Cl.: A61F 2/02, A61F 2/08, A61L 27/54

(54) **PLATFORM FOR ENGINEERED IMPLANTABLE TISSUES AND ORGANS AND METHODS OF MAKING THE SAME**
PLATTFORM FÜR VERÄNDERTE IMPLANTIERBARE GEWEBE UND ORGANE SOWIE HERSTELLUNGSVERFAHREN DAFÜR
PLATEFORME POUR DES TISSUS ET ORGANES ARTIFICIELS IMPLANTABLES ET PROCÉDÉS DE FABRICATION DE CES TISSUS ET ORGANES

(30) Priority: 12.09.2011 US 201161533766 P; 12.09.2011 US 201161533761 P
(43) Date of publication of application: 23.07.2014
(73) Proprietor: Organovo, Inc., San Diego, CA 92121 (US)
(72) Inventor: MURPHY, Keith, Palos Verdes Estates, CA 90274 (US); KHATIWALA, Chirag, San Diego, CA 92126 (US); DORFMAN, Scott, Baltimore, MD 21231 (US); SHEPHERD, Benjamin, San Diego, CA 92131 (US); PRESNELL, Sharon, San Diego, CA 92128 (US)
(74) Representative: HGF Limited
(86) International application number: PCT/US2012/054935
(87) International publication number: WO 2013/040087

(56) References cited:
- WO-A2-2005/081970
- WO-A2-2010/008905
- WO-A2-2011/038373
- WO-A2-2012/003465
- US-A1- 2004 132 184
- US-A1- 2008 193 910
- US-A1- 2009 142 307
- KAROLY JAKAB ET AL: "Tissue Engineering by Self-Assembly of Cells Printed into Topologically Defined Structures", TISSUE ENGINEERING PART A, vol. 14, no. 3, 1 March 2008 (2008-03-01), pages 413-421, XP55047480, ISSN: 1937-3341, DOI: 10.1089/tea.2007.0173
- DATABASE BIOSIS [Online] BIOSCIENCES INFORMATION SERVICE, PHILADELPHIA, PA, US; April 2007 (2007-04), XU TAO ET AL: "Bio-printing of living organized tissues using an inkjet technology", XP002737973, Database accession no. PREV200700335042 & FASEB JOURNAL, vol. 21, no. 5, April 2007 (2007-04), page A636, EXPERIMENTAL BIOLOGY 2007 ANNUAL MEETING; WASHINGTON, DC, USA; APRIL 28 -MAY 02, 2007 ISSN: 0892-6638
- XU T ET AL: "In vivo generation of functional tissues using the inkjet printing technology", TISSUE ENGINEERING, LARCHMONT, NY, US, vol. 13, no. 7, 7 September 2007 (2007-09-07), pages 1713-1714, XP009135596, ISSN: 1076-3279
- SANGJUN MOON ET AL: "Layer by Layer Three-dimensional Tissue Epitaxy by Cell-Laden Hydrogel Droplets", TISSUE ENGINEERING PART C: METHODS, vol. 16, no. 1, 1 February 2010 (2010-02-01), pages 157-166, XP055180383, ISSN: 1937-3384, DOI: 10.1089/ten.tec.2009.0179
- FUELLHASE C ET AL: "264 GENERATION OF ORGANIZED BLADDER TISUE CONSTRUCTS USING A NOVEL HYBRID PRINTING SYSTEM", EUROPEAN UROLOGY SUPPLEMENTS,, vol. 8, no. 4, 1 March 2009 (2009-03-01), page 186, XP026061648, ISSN: 1569-9056, DOI: 10.1016/S1569-9056(09)60269-4 [retrieved on 2009-03-01]
- JAKAB KAROLY ET AL: "Tissue engineering by self-assembly and bio-printing of living cells", BIOFABRICATION, INSTITUTE OF PHYSICS PUBLISHING LTD, UK, vol. 2, no. 2, 2 June 2010 (2010-06-02), pages 1-14, XP003032856, ISSN: 1758-5082
- NOROTTE, C. ET AL.: 'Scaffold-free vascular tissue engineering using bioprinting' BIOMATERIALS vol. 30, 2009, pages 5910 - 5917, XP026524655
- FURUKAWA, K. S. ET AL.: 'Scaffold-free cartilage tissue by mechanical stress loading for tissue engineering' TISSUE ENGINEERING. 2010, pages 409 - 428, XP055147923

## Description

### BACKGROUND OF THE INVENTION

A number of pressing problems confront the healthcare industry. As of December 2009 there were 105,305 patients registered by United Network for Organ Sharing (UNOS) as needing an organ transplant. Between January and September 2009, only 21,423 transplants were performed. Each year more patients are added to the UNOS list than transplants are performed, resulting in a net increase in the number of patients waiting for a transplant. For example, at the beginning of 2008, 75,834 people were registered as needing a kidney; at the end of that year, the number had grown to 80,972. 16,546 kidney transplants were performed that year, but 33,005 new patients were added to the list. The 2008 transplant rate for a patient registered by UNOS as needing a kidney was 20%. The mortality rate of waitlist patients was 7%.

SangJun Moon et al. disclose bioengineered, multilayered muscle tissue patches prepared by bioprinting ["Layer be Layer Three-Dimensional Tissue Epitaxy by Cell-Laden Hydrogel Droplets", TISSUE ENGINEERING: Part C, Vol. 16 (1), 2010; XP055180383].

### SUMMARY OF THE INVENTION

There is a need for materials, tools, and techniques that facilitate application of regenerative medicine and tissue engineering technologies to relieving the urgent need for implantable tissues and organs. Moreover, there is a need for implantable tissues and organs that are suitable for wound repair, tissue augmentation, organ repair, and organ replacement. Accordingly, the inventors describe herein implantable tissues, organs, and methods of making the same.

The scope of the invention is defined by the claims. Any references in the description to methods of treatment refer to the compositions/tissues/organs of the present invention for use in a method for treatment of the human (or animal) body by therapy.

According to the invention, disclosed herein are living, three-dimensional engineered tissues or organs comprising a plurality of layers, the plurality of layers characterized by being a) substantially free of pre-formed scaffold at the time of bioprinting and at the time of use; and b) having at least one component bioprinted by extrusion of a bio-ink comprising a plurality of cells, the bio-ink being a solid or semi-solid composition, onto a biocompatible surface; the plurality of layers suitable for implantation in a vertebrate subject upon sufficient maturation; provided that at least one layer of the engineered tissue or organ comprises muscle cells and that the engineered tissue or organ is a sheet and is not a vascular tube, and that the plurality of layers are cohered to one another. In some embodiments, at least one layer comprises a plurality of cell types, the cell types spatially arranged relative to each other to create a planar geometry. In further embodiments, at least one layer is at least 100 µm thick in its smallest dimension at the time of fabrication. In some embodiments, the tissue or organ comprises a plurality of layers, at least one layer compositionally or architecturally distinct from at least one other layer to create a laminar geometry. In further embodiments, at least one layer is at least 100 µm thick in its smallest dimension at the time of fabrication. The tissue or organ is a sheet and is not a vascular tube. The tissue or organ is substantially free of any pre-formed scaffold at the time of bioprinting and at the time of use. The tissue or organ is bioprinted. In some embodiments, one or more layers generates an extracellular matrix. In some embodiments, the muscle cells are smooth muscle cells. In some embodiments, the muscle cells are skeletal muscle cells. In some embodiments, the muscle cells are cardiac muscle cells. In some embodiments, the muscle cells were derived from stem cells or progenitor cells capable of differentiating into the muscle cells. In further embodiments, the stem cells or progenitor cells were differentiated into the muscle cells before, during, or after fabrication. In some embodiments, the tissue or organ further comprises cells selected from: endothelial cells, nerve cells, pericytes, fibroblasts, tissue-specific epithelial cells, chondrocytes, skeletal muscle cells, cardiomyocytes, bone-derived cells, soft tissue-derived cells, mesothelial cells, tissue-specific stromal cells, stem cells, progenitor cells, endoderm-derived cells, ectoderm-derived cells, mesoderm-derived cells, and combinations thereof. In some embodiments, cells other than muscle cells were dispensed on at least one surface of the one or more layers. In further embodiments, cells other than muscle cells were bioprinted on at least one surface of the one or more layers. In some embodiments, the cells other than muscle cells were dispensed on the one or more layers at substantially the same time the one or more layers was fabricated, following fabrication of the one or more layers, during maturation of the one or more layers, or following maturation of the one or more layers. In some embodiments, cells other than muscle cells were dispensed on the one or more layers as a layer of cells about 1 to about 20 cells thick. In some embodiments, the one or more layers are substantially planar. In further embodiments, the tissue or organ is a muscle cell-comprising sheet or patch suitable for wound repair, tissue replacement, or tissue augmentation.

In another aspect, disclosed herein is implantation of the tissues and/or organs.

In another aspect, disclosed herein is maintenance of the tissues and/or organs in culture for *ex-vivo* research use.

According to the invention, disclosed herein are methods for making a three dimensional, engineered, implantable tissue or organ comprising a plurality of layers, comprising a muscle cell-containing layer, the method comprising: bioprinting at least one component by extruding bio-ink which is a solid or semi-solid composition comprising muscle cells into a form onto a biocompatible surface; and fusing the bio-ink into a cohesive cellular structure; wherein the implantable tissue or organ is substantially free of any pre-formed scaffold at the time of bioprinting and at the time of use, and provided that the tissue or organ is implantable in a vertebrate subject, is a sheet and and not a vascular tube, and the plurality of layers are cohered to one another. The implantable tissue or organ is substantially free of any pre-formed scaffold at the time bioprinting and at the time of use. In some embodiments, the muscle cells are smooth muscle cells. In some embodiments, the muscle cells are skeletal muscle cells. In some embodiments, the muscle cells are cardiac muscle cells. In some embodiments, the muscle cells are differentiated from progenitors. In some embodiments, the muscle cells are generated from a tissue sample. In further embodiments, the tissue sample is lipoaspirate. The form is a sheet. In some embodiments, the bio-ink further comprises cells selected from: endothelial cells, nerve cells, pericytes, fibroblasts, tissue-specific epithelial cells, chondrocytes, skeletal muscle cells, cardiomyocytes, bone-derived cells, soft tissue-derived cells, mesothelial cells, tissue-specific stromal cells, stem cells, progenitor cells, endoderm-derived cells, ectoderm-derived cells, mesoderm-derived cells, and combinations thereof. In some embodiments, the method further comprises the step of bioprinting, spraying, painting, applying, dip coating, grafting, seeding, injecting, or layering cells other than muscle cells into or onto the bioprinted form. In some embodiments, the method further comprises bioprinting, spraying, painting, applying, dip coating, grafting, injecting, seeding, or layering cells other than muscle cells into or onto the cohesive cellular structure.

In another aspect, disclosed herein are living, three-dimensional engineered tissues or organs comprising a plurality of layers, the plurality of layers characterized by a) substantially scaffold-free at the time of bioprinting and at the time of use; and b) bioprinted, the one or more layers matured into implantation-ready status for a vertebrate subject; the engineered tissue or organ consisting essentially of cellular material; provided that at least one layer of the engineered tissue or organ comprises muscle cells and that the engineered tissue or organ is a sheet and is not a vascular tube. In some embodiments, at least one layer comprises a plurality of cell types, the cell types spatially arranged relative to each other to create a planar geometry. In further embodiments, at least one layer is at least 100 µm thick in its smallest dimension at the time of fabrication. The tissue or organ comprises a plurality of layers, at least one layer compositionally or architecturally distinct from at least one other layer to create a laminar geometry. In further embodiments, at least one layer is at least 100 µm thick in its smallest dimension at the time of fabrication. The tissue or organ is a sheet and is not a vascular tube. In some embodiments, the muscle cells are smooth muscle cells. In some embodiments, the muscle cells are skeletal muscle cells. In some embodiments, the muscle cells are cardiac muscle cells. In some embodiments, the tissue or organ further comprises cells selected from: endothelial cells, nerve cells, pericytes, fibroblasts, tissue-specific epithelial cells, chondrocytes, skeletal muscle cells, cardiomyocytes, bone-derived cells, soft tissue-derived cells, mesothelial cells, tissue-specific stromal cells, stem cells, progenitor cells, endoderm-derived cells, ectoderm-derived cells, mesoderm-derived cells, and combinations thereof. In some embodiments, cells are dispensed on at least one surface of the at least one layer. In further embodiments, cells are bioprinted on at least one surface of the at least one layer.

In another aspect, disclosed herein is implantation of the tissues and/or organs.

In another aspect, disclosed herein is maintenance of the tissues and/or organs in culture for *ex-vivo* research use.

### BRIEF DESCRIPTION OF THE DRAWINGS

The novel features of the invention are set forth in the appended claims. A better understanding of the features and advantages of the present invention will be obtained by reference to the following detailed description that sets forth illustrative embodiments, and the accompanying drawings of which:

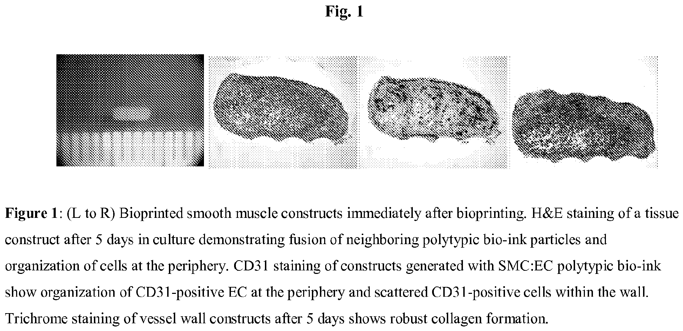

**Fig. 1** depicts non-limiting examples of bioprinted smooth muscle patches (e.g., sheets), constructed with bio-ink comprised of smooth muscle cells (SMC) and also containing endothelial cells (EC). In this example, the bio-ink was configured in a cylindrical format prior to bioprinting. Various histologic stains are shown to indicate distribution and position of cell types.

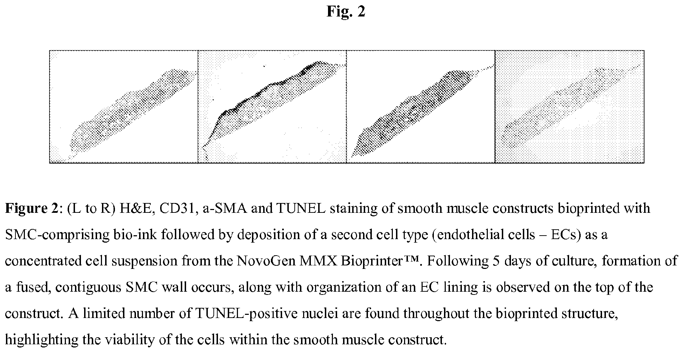

**Fig. 2** depicts non-limiting examples of bioprinted planar smooth muscle patches (e.g., sheets), constructed with bio-ink comprised solely of SMC. In this example, the SMC bio-ink was free of any scaffold or exogenously added biomaterial and was bioprinted on the NovoGen MMX bioprinter using a cylindrical bioprinting format. In this example, a second cell type (endothelial cells) was bioprinted as a thin layer on a single surface of the bioprinted SMC patch immediately after fabrication. Various histological stains are shown to indicate distribution and position of cell types.

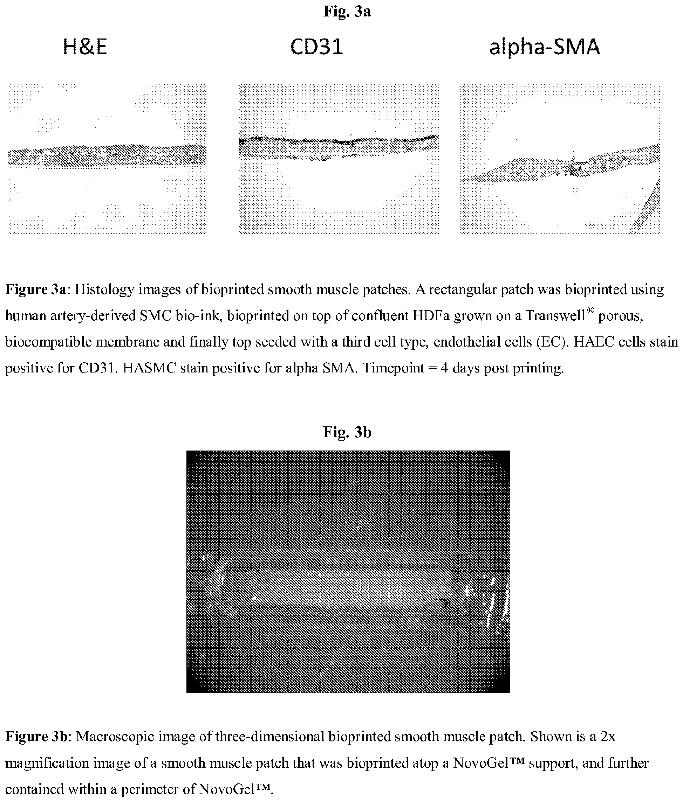

**Fig. 3a** depicts non-limiting examples of bioprinted planar smooth muscle patches (e.g., sheets) out of bio-ink that consisted of human artery-derived SMCs. In this example, the SMCs were printed on top of a layer of human dermal fibroblasts (HDF) to mimic the native biology of a smooth muscle cell layer adjacent to a fibroblast-comprising adventitia. In this example, a third cell type (human artery-derived endothelial cells) was bioprinted as a thin layer atop the bioprinted smooth muscle patch. HASMC are stained for alpha SMA.
**Fig. 3b** is a macroscopic image depicting a non-limiting example of a smooth muscle patch (composed of SMC bio-ink), shown immediately after bioprinting on the NovoGen MMX bioprinter. In this example, a non-adherent hydrogel confinement material (NovoGel™) was utilized as a base support onto which the construct was printed, as well as a confinement window around the bioprinted smooth muscle patch.

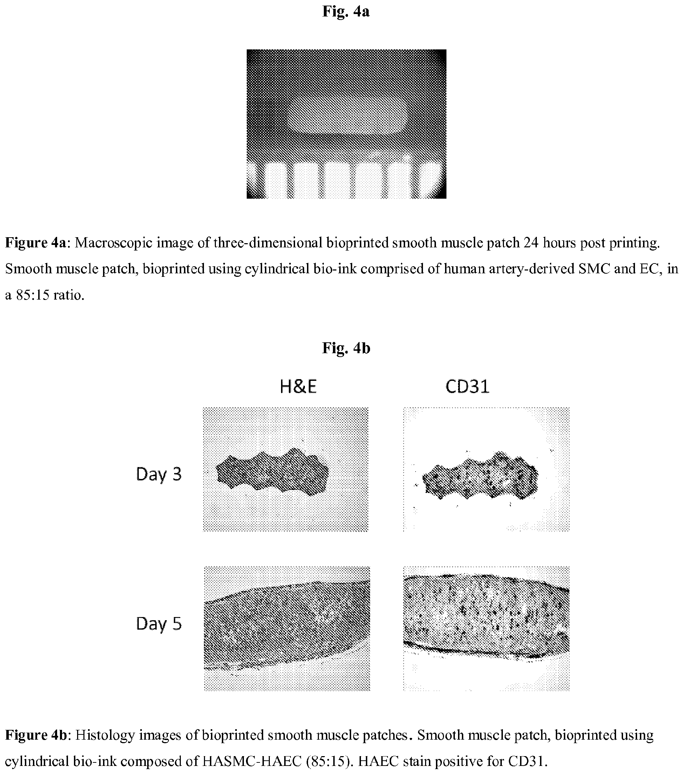

**Fig. 4a** is a macroscopic image depicting a non-limiting example of a bioprinted planar smooth muscle patches (e.g., sheets) constructed with cylindrical bio-ink comprised of human artery-derived SMCs in combination with human artery-derived endothelial cells, mixed at a ratio of 85:15.
**Fig. 4b** depicts non-limiting examples of bioprinted planar smooth muscle patches (e.g., sheets) constructed with cylindrical bio-ink comprised of SMC:EC at a ratio of 85:15. The EC (endothelial cells) were identified by immunostaining for CD31, a specific marker of endothelial cells.

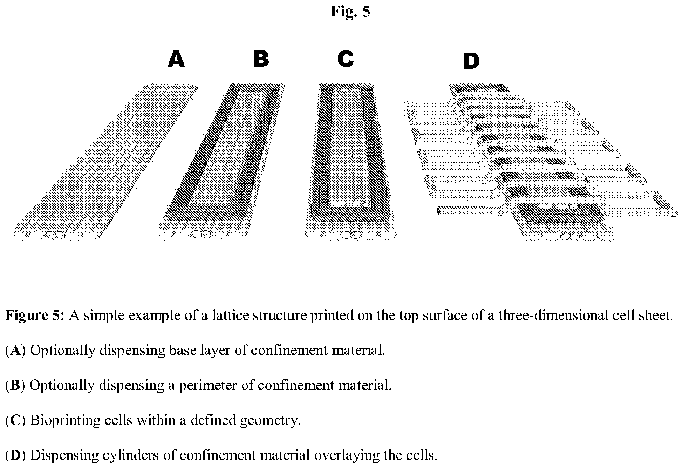

**Fig. 5** is a non-limiting example of a bioprinted smooth muscle sheet that has been bioprinted within a non-adherent hydrogel support structure, wherein the confinement material placed on top of the bioprinted smooth muscle patch is configured in a lattice structure to allow direct contact with at least some portion(s) of the bioprinted sheet and a nutrient media; also depicted are exemplary steps for fabricating the same.

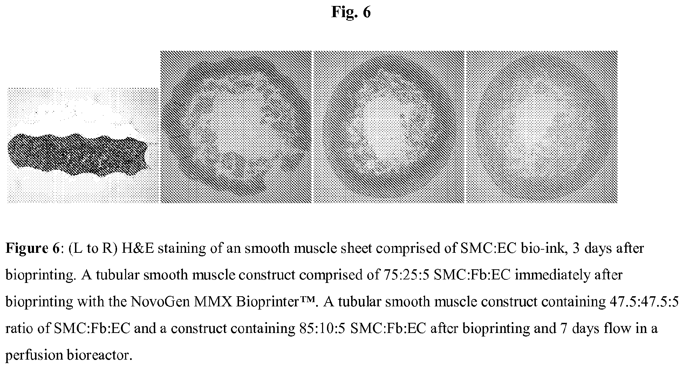

**Fig. 6** is a non-limiting example (not forming part of the invention) of bioprinted smooth muscle-comprising tube. In this example, the bio-ink comprised SMC combined with two additional cell types (fibroblasts and endothelial cells) at ratios of 75:25:5, 47.5:47.5:5, and 85:10:5, from left to right.

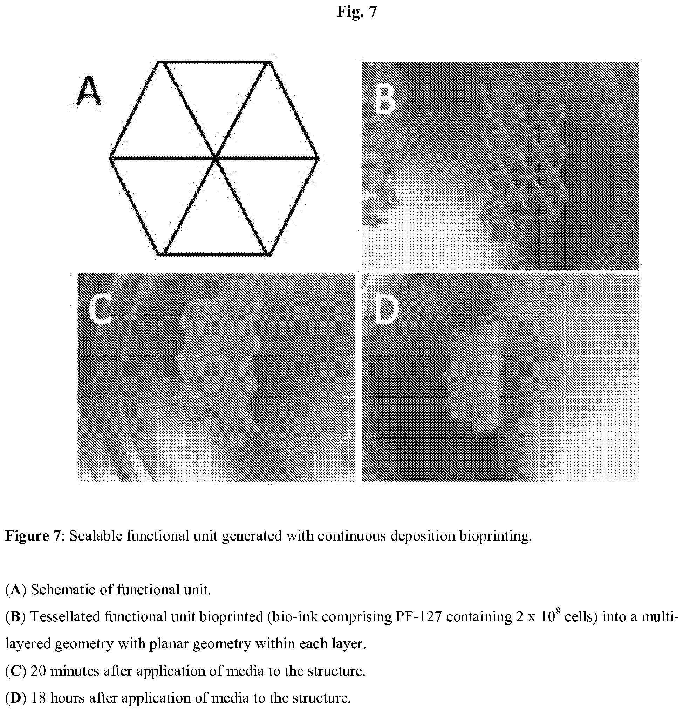

**Fig. 7** is a macroscopic image depicting a non-limiting example of an engineered liver tissue, in this case, a liver tissue bioprinted using a continuous deposition mechanism using bio-ink composed of cells encapsulated in an extrusion compound (e.g., PF-127). (A) shows a schematic diagram of a single functional unit; **(B)** a multi-layer sheet with planar, tessellated geometry in each layer; **(C)** and **(D)** show the construct after application of media and dissolution of the extrusion compound.

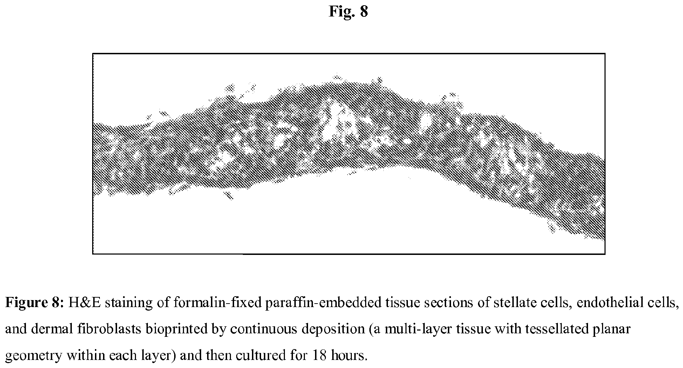

**Fig. 8** is a photomicrograph of the H&E stained multi-layered construct of **Fig. 7****,** depicting an exemplary "spoke" in the tessellated geometry.

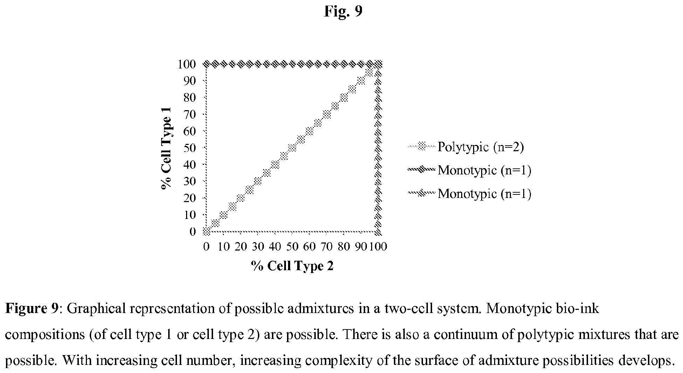

**Fig. 9** is a line graph illustrating possible admixtures in a two-cell system.

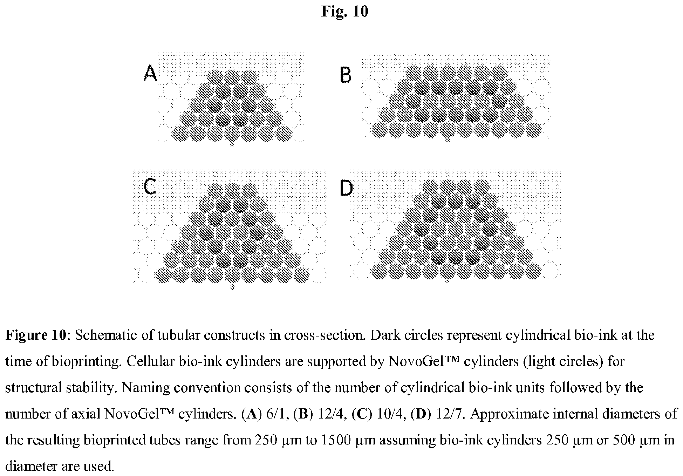

**Fig. 10** is a schematic diagram of tubular constructs in cross section (not forming part of the invention). An exemplary naming convention consists of the number of cylindrical bio-ink units followed by the number of axial NovoGel™ cylinders **(A)** 6/1, **(B)** 12/4, **(C)** 10/4, and **(D)** 12/7.

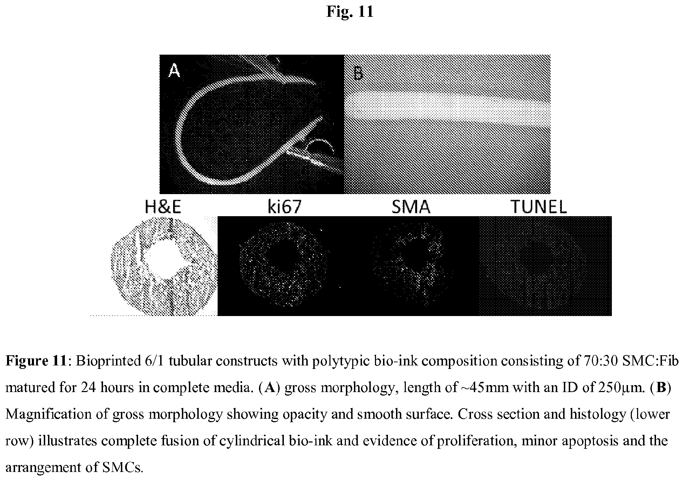

**Fig. 11** depicts a bioprinted 6/1 tubular constructs (not forming part of the invention) with polytypic bio-ink composition including **(A)** macroscopic gross morphology, **(B)** magnification of gross morphology showing opacity and smooth surface, and photomicrographs of cross sectional histology (lower row).

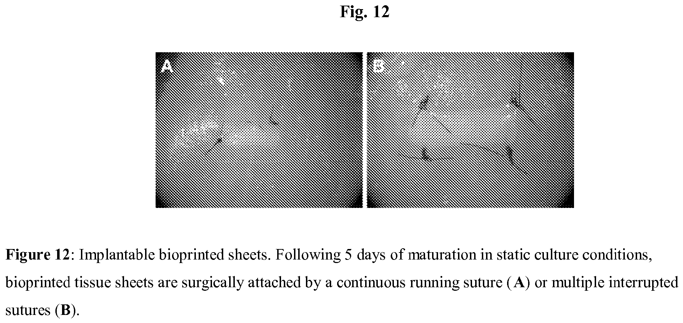

**Fig. 12** illustrates bioprinted tissue sheets surgically attached by a continuous running suture **(A)** or multiple interrupted sutures **(B).**

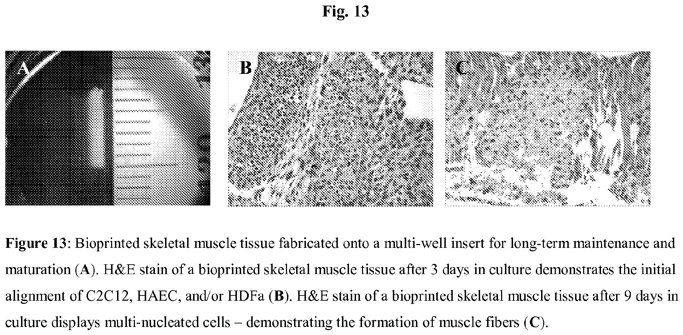

**Fig. 13** depicts a bioprinted skeletal muscle tissue fabricated onto a multi-well insert for long-term maintenance and maturation **(A).** H&E stain of a bioprinted skeletal muscle tissue after 3 days and 9 days in culture **(B, C**).

### DETAILED DESCRIPTION OF THE INVENTION

The invention relates to the field of regenerative medicine and tissue/organ engineering. More particularly, the invention relates to tissues and organs comprising at least one layer comprising muscle cells, wherein the engineered tissue or organ consists essentially of cellular material, and methods of making the same. An advantage of the tissues, organs, and methods disclosed herein include, by way of example, flexible three-dimensional tissue geometry that allows fabrication of layered sheets comprising muscle cells. Another advantage is a flexible layered approach allowing for one or more cell types other than muscle cells to be disposed, dispensed, and/or bioprinted on at least one surface of the layer. These advantages result in engineered tissues and organs that mimic native tissue composition and architecture.

According to the invention, disclosed are living, three-dimensional engineered tissues or organs comprising a plurality of layers, the plurality of layers characterized by being: a) substantially free of pre-formed scaffold at the time of bioprinting and at the time of use; and b) having at least one component bioprinted by extrusion of a bio-ink comprising a plurality of cells, the bio-ink being a solid or semi-solid composition, onto a biocompatible surface, the plurality of layers suitable for implantation in a vertebrate subject upon sufficient maturation; provided that at least one layer of the engineered tissue or organ comprises muscle cells and that the engineered tissue or organ is a sheet and is not a vascular tube, and the plurality of layers are cohered to one another.

Also disclosed herein, in certain embodiments, is implantation of the tissues and/or organs.

According to the invention also disclosed are methods for making a three-dimensional, engineered, implantable tissue or organ comprising a plurality of layers, comprising a muscle cell-containing layer, the method comprising: bioprinting at least one component by extruding bio-ink which is a solid or semi-solid composition comprising muscle cells into a form onto a biocompatible surface; and fusing the bio-ink into a cohesive cellular structure; wherein the implantable tissue or organ is substantially free of any pre-formed scaffold at the time of bioprinting and at the time of use, and provided that the tissue or organ is implantable in a vertebrate subject, is a sheet and not a vascular tube, and the plurality of layers are cohered to one another.

Also disclosed herein, in certain embodiments, are living, three-dimensional engineered tissues or organs comprising a plurality of layers, the plurality of layers characterized by one or more of: a) substantially free of pre-formed scaffold at the time of bioprinting and at the time of use; and b) bioprinted, the one or more layers matured into implantation-ready status for a vertebrate subject; the engineered tissue or organ consisting essentially of cellular material; provided that at least one layer of the engineered tissue or organ comprises muscle cells and that the engineered tissue or organ is a sheet and is not a vascular tube.

Also disclosed herein, in certain embodiments, is implantation of the tissues and/or organs.

### Certain Definitions

Unless otherwise defined, all technical and scientific terms used herein have the same meaning as commonly understood by one of ordinary skill in the art to which this invention belongs.

As used in this specification and the appended claims, the singular forms "a," "an," and "the" include plural references unless the context clearly dictates otherwise. Thus, for example, references to "a nucleic acid" includes one or more nucleic acids, and/or compositions of the type described herein which will become apparent to those persons skilled in the art upon reading this disclosure and so forth. Any reference to "or" herein is intended to encompass "and/or" unless otherwise stated.

As used herein, "bio-ink" means a semi-solid, or solid composition comprising a plurality of cells. In some embodiments, bio-ink comprises cell solutions, cell aggregates, cell-comprising gels, multicellular bodies, or tissues. In some embodiments, the bio-ink additionally comprises support material. In some embodiments, the bio-ink additionally comprises non-cellular materials that provide specific biomechanical properties that enable bioprinting.

As used herein, "bioprinting" means utilizing three-dimensional, precise deposition of cells (e.g., cell solutions, cell-containing gels, cell suspensions, cell concentrations, multicellular aggregates, multicellular bodies, etc.) via methodology that is compatible with an automated or semi-automated, computer-aided, three-dimensional prototyping device (e.g., a bioprinter).

As used herein, "blood vessel" means a tube of smooth muscle cells further comprising vascular endothelial cells, and having an internal diameter greater than 100 µm, and intended for use *in vivo* as an interpositional vascular graft, a bypass vascular graft, or an arterio-venous vascular shunt. As used herein, "blood vessel" expressly does not include the integral vascular components (arteries, veins, arterioles, venules, capillaries, and microvasculature) of other organs or tissues. For example, the vascular network associated with the bladder, intestine, or esophagus would not be included in the definition of "blood vessel" as presented herein.

As used herein, "cohere," "cohered," and "cohesion" refer to cell-cell adhesion properties that bind cells, cell aggregates, multicellular aggregates, multicellular bodies, and/or layers thereof. The terms are used interchangeably with "fuse," "fused," and "fusion."

As used herein, "extracellular matrix" means proteins that are produced by cells and transported out of the cells into the extracellular space, where they serve as a support to hold tissues together, to provide tensile strength, and/or to facilitate cell signaling.

As used herein, "implantable" means biocompatible and capable of being inserted or grafted into or affixed onto a living organism either temporarily or substantially permanently.

As used herein, "laminar" means a multi-layered bioprinted tissue in which two or more planar layers are combined to increase the overall thickness of the tissue in the z-plane. In some embodiments, each planar layer is substantially similar in architecture and/or composition. In other embodiments, each planar layer is substantially distinct in architecture and/or composition.

As used herein, "multi-layered" means being comprised of two or more layers of tissue, wherein each tissue layer is one or more cell-layers in thickness. In some embodiments, layers of tissue are deposited one at a time. In other embodiments, multiple layers are deposited simultaneously. Optionally, each layer is comprised of multiple cell types. Further, the multiple cell types within each layer are optionally arranged relative to each other in a spatially-defined architecture in the x-y planes (i.e., horizontal planes). Furthermore, addition of layers in the z-plane (i.e., vertical plane), in some cases, results in controlled spatial positioning of the cells within the layers relative to each other so that a spatially-defined architecture is continued in the z-plane.

As used herein, "organ" means a collection of tissues joined into structural unit to serve a common function. Examples of organs include, but are not limited to, skin, urethra, conduit, ureter, bladder, fallopian tube, uterus, trachea, bronchus, lymphatic vessel, esophagus, stomach, gallbladder, small intestine, large intestine, and colon.

As used herein, "planar" means a layer of multicellular bioprinted tissue in which multiple bio-ink compositions and/or void spaces are spatially arranged into a defined pattern relative to each other within the x-y plane of the tissue layer. "Planar" also means substantially flat when used to describe the shape of a tissue "sheet" or "patch."

As used herein, "scaffold" refers to synthetic scaffolds such as polymer scaffolds and porous hydrogels, non-synthetic scaffolds such as pre-formed extracellular matrix layers, dead cell layers, and decellularized tissues, and any other type of pre-formed scaffold that is integral to the physical structure of the engineered tissue and/or organ and not able to be removed from the tissue and/or organ without damage/destruction of said tissue and/or organ. In further embodiments, decellularized tissue scaffolds include decellularized native tissues or decellularized cellular material generated by cultured cells in any manner; for example, cell layers that are allowed to die or are decellularized, leaving behind the ECM they produced while living. The term "scaffold-free" as used herein indicates that the cell-comprising tube, sac, or sheet is substantially free from scaffold (as defined above) at the time of use. "Scaffold-free" is used interchangeably with "scaffoldless" and "free of pre-formed scaffold."

As used herein, "stem cell" means a cell that exhibits potency and self-renewal. Stem cells include, but are not limited to, totipotent cells, pluripotent cells, multipotent cells, oligopotent cells, unipotent cells, and progenitor cells. In various embodiments, stem cells are embryonic stem cells, peri-natal stem cells, adult stem cells, amniotic stem cells, and induced pluripotent stem cells.

As used herein, "subject" means any individual. The term is interchangeable with "patient," "recipient," and "donor." None of the terms should be construed as requiring the supervision (constant or otherwise) of a medical professional (e.g., physician, nurse, nurse practitioner, physician's assistant, orderly, hospice worker, social worker and a clinical research associate) or a scientific researcher.

As used herein, "tissue" means an aggregate of cells. Examples of tissues include, but are not limited to, connective tissue (e.g., areolar connective tissue, dense connective tissue, elastic tissue, reticular connective tissue, and adipose tissue), muscle tissue (e.g., skeletal muscle, smooth muscle and cardiac muscle), genitourinary tissue, gastrointestinal tissue, pulmonary tissue, bone tissue, nervous tissue, and epithelial tissue (e.g., simple epithelium and stratified epithelium), endoderm-derived tissue, mesoderm-derived tissue, and ectoderm-derived tissue.

### Tissue Engineering

Tissue engineering is an interdisciplinary field that applies and combines the principles of engineering and life sciences toward the development of biological substitutes that restore, maintain, or improve tissue function through augmentation, repair, or replacement of an organ or tissue. The basic approach to classical tissue engineering is to seed living cells into a biocompatible and eventually biodegradable environment (e.g., a scaffold), and then culture this construct in a bioreactor so that the initial cell population expands further and mature to generate the target tissue upon implantation. With an appropriate scaffold that mimics the biological extracellular matrix (ECM), the developing tissue adopts both the form and function of the desired organ after *in vitro* and *in vivo* maturation. However, achieving high enough cell density with a native tissue-like architecture is challenging due to the limited ability to control the distribution and spatial arrangement of the cells throughout the scaffold. These limitations often result in tissues or organs with poor mechanical properties and/or insufficient function. Additional challenges exist with regard to biodegradation of the scaffold, entrapment of residual polymer, and industrial scale-up of manufacturing processes. Scaffoldless approaches have been attempted. Current scaffoldless approaches are subject to several limitations:
- Complex geometries, such as multi-layered structures wherein each layer comprises a different cell type, often require definitive, high-resolution placement of cell types within a specific architecture to reproducibly achieve a native tissue-like outcome.
- Scale and geometry are limited by diffusion and/or the requirement for functional vascular networks for nutrient supply.
- The viability of the tissues in many cases is compromised by confinement material that limits diffusion and restricts the cells' access to nutrients.

Disclosed herein, in certain embodiments, are engineered tissues and organs, and methods of fabrication. The tissue engineering methods disclosed herein have the following advantages:
- They are capable of producing cell-comprising tissues and/or organs.
- They mimic the environmental conditions found within the development, homeostasis, and/or pathogenesis of natural tissues by re-creating native tissue-like intercellular interactions.
- They optionally achieve a broad array of complex topologies (e.g., multilayered structures, segments, sheets, tubes, sacs, etc.).
- They are compatible with automated means of manufacturing and are scalable.

Bioprinting enables improved methods of generating cell-comprising implantable tissues that are useful in tissue repair, tissue augmentation, tissue replacement, and organ replacement (see below).

### Bioprinting

In some embodiments, at least one component of the engineered, implantable tissues and/or organs was bioprinted. In further embodiments, the engineered, implantable tissues and/or organs were entirely bioprinted. In still further embodiments, bioprinted constructs are made with a method that utilizes a rapid prototyping technology based on three-dimensional, automated, computer-aided deposition of cells, including cell solutions, cell suspensions, cell-comprising gels or pastes, cell concentrations, multicellular bodies (e.g., cylinders, spheroids, ribbons, etc.), and confinement material onto a biocompatible surface (e.g., composed of hydrogel and/or a porous membrane) by a three-dimensional delivery device (e.g., a bioprinter). As used herein, in some embodiments, the term "engineered," when used to refer to tissues and/or organs means that cells, cell solutions, cell suspensions, cell-comprising gels or pastes, cell concentrates, multicellular aggregates, and layers thereof are positioned to form three-dimensional structures by a computer-aided device (e.g., a bioprinter) according to a computer script. In further embodiments, the computer script is, for example, one or more computer programs, computer applications, or computer modules. In still further embodiments, three-dimensional tissue structures form through the post-printing fusion of cells or multicellular bodies similar to self-assembly phenomena in early morphogenesis.

While a number of methods are available to arrange cells, multicellular aggregates, and/or layers thereof on a biocompatible surface to produce a three-dimensional structure including manual placement, positioning by an automated, computer-aided machine such as a bioprinter is advantageous. Advantages of delivery of cells or multicellular bodies with this technology include rapid, accurate, and reproducible placement of cells or multicellular bodies to produce constructs exhibiting planned or pre-determined orientations or patterns of cells, multicellular aggregates and/or layers thereof with various compositions. Advantages also include assured high cell density, while minimizing cell damage.

In some embodiments, the method of bioprinting is continuous and/or substantially continuous. A non-limiting example of a continuous bioprinting method is to dispense bio-ink from a bioprinter via a dispense tip (e.g., a syringe, capillary tube, etc.) connected to a reservoir of bio-ink. In further non-limiting embodiments, a continuous bioprinting method is to dispense bio-ink in a repeating pattern of functional units. In various embodiments, a repeating functional unit has any suitable geometry, including, for example, circles, squares, rectangles, triangles, polygons, and irregular geometries. In further embodiments, a repeating pattern of bioprinted function units comprises a layer and a plurality of layers are bioprinted adjacently (e.g., stacked) to form an engineered tissue or organ. In various embodiments, 2, 3, 4, 5, 6, 7, 8, 9, 10, 11, 12, 13, 14, 15, or more layers are bioprinted adjacently (e.g., stacked) to form an engineered tissue or organ.

In some embodiments, a bioprinted functional unit repeats in a tessellated pattern. A "tessellated pattern" is a plane of figures that fills the plane with no overlaps and no gaps. **Fig. 7A** shows an example of a functional unit that is optionally repeated to produce the tessellation pattern depicted in **Fig. 7B****.** Advantages of continuous and/or tessellated bioprinting include, by way of non-limiting example, increased productivity of bioprinted tissue. Another non-limiting, exemplary advantage is eliminating the need to align the bioprinter with previously deposited elements of bio-ink. Continuous bioprinting also facilitates printing larger tissues from a large reservoir of bio-ink, optionally using a syringe mechanism.

In various embodiments, methods in continuous bioprinting involves optimizing and/or balancing parameters such as print height, pump speed, robot speed, or combinations thereof independently or relative to each other. In one example, the bioprinter head speed for deposition was 3 mm/s, with a dispense height of 0.5 mm for the first layer and dispense height was increased 0.4 mm for each subsequent layer. In some embodiments, the dispense height is approximately equal to the diameter of the bioprinter dispense tip. Without limitation a suitable and/or optimal dispense distance does not result in material flattening or adhering to the dispensing needle. In various embodiments, the bioprinter dispense tip has an inner diameter of about, 20, 50, 100, 150, 200, 250, 300, 350, 400, 450, 500, 550, 600, 650, 700, 750, 800, 850, 900, 950, 1000 µm, or more, including increments therein. In various embodiments, the bio-ink reservoir of the bioprinter has a volume of about .5, 1, 2, 3, 4, 5, 6, 7, 8, 9, 10, 15, 20, 25, 30, 35, 40, 45, 50, 55, 60, 65, 70, 75, 80, 85, 90, 95, 100 cubic centimeters, or more, including increments therein. In some embodiments, the pump speed is suitable and/or optimal when the residual pressure build-up in the system is low. In some embodiments, favorable pump speeds depend on the ratio between the cross-sectional areas of the reservoir and dispense needle with larger ratios requiring lower pump speeds. In some embodiments, a suitable and/or optimal print speed enables the deposition of a uniform line without affecting the mechanical integrity of the material.

Disclosed are business methods (not forming art of the invention). In some embodiments, the speed and scalability of the techniques and methods disclosed herein are utilized to design, build, and operate industrial and/or commercial facilities for production of engineered tissues and/or organs for implantation. In further embodiments, the engineered tissues and/or organs are produced, stored, distributed, marketed, advertised, and sold as, for example, materials, tools, and kits for *in vivo* uses such as medical treatment of tissue damage, tissue disease, and/or organ failure. In other embodiments, the engineered tissues and/or organs are produced, stored, distributed, marketed, advertised, and sold as, for example, materials, tools, and kits for *in vitro* uses such as scientific and/or medical research. In further embodiments, the engineered tissues and/or organs are maintained in cell culture environments and used in scientific and/or medical research.

### Engineered tissues and organs

Disclosed herein, in certain embodiments, are engineered, implantable tissues and/or organs comprising a plurality of layers, wherein at least one layer comprises muscle cells. The plurality of layers are characterized by being substantially free of pre-formed scaffold at the time of use and at the time of bioprinting (a technology described herein). In further embodiments, the plurality of layers and/or the engineered tissue or organ consist essentially of cellular material. The plurality of layers are suitable for implantation in a vertebrate subject upon sufficient maturation. In some embodiments, the plurality of layers are matured into implantation-ready status for a vertebrate subject.

In some embodiments, the engineered tissues and organs consist essentially of cellular material. In various further embodiments, the cell-comprising portions of the engineered tissues and organs consist of 30, 35, 40, 45, 50, 55, 60, 65, 70, 75, 80, 85, 90, 95, 96, 97, 98, 99, 99.5, 99.9, and 100% cellular material, including increments therein, at the time of construction. In other various embodiments, the cell-comprising portions of the engineered tissues and organs consist of 30, 35, 40, 45, 50, 55, 60, 65, 70, 75, 80, 85, 90, 95, 96, 97, 98, 99, 99.5, 99.9, and 100% cellular material, including increments therein, at the time of use. In some embodiments, the engineered tissues are cohered and/or adhered aggregates of cells. In some embodiments, the non-cellular components are removed prior to use. In further embodiments, the non-cellular components are removed by physical, chemical, or enzymatic means. In some embodiments, a proportion of the non-cellular components remains associated with the cellular components at the time of use. In some embodiments, the non-cellular components are selected from a group that includes: hydrogels, surfactant polyols, thermo-responsive polymers, hyaluronates, alginates, collagens, or other biocompatible natural or synthetic polymers.

The engineered tissues optionally mimic any human or mammalian tissue. Exemplary tissues include epithelial tissue, connective tissue, muscle tissue, nervous tissue, and the like. In some embodiments, the engineered organs are collections of tissues joined into structural unit(s) to serve a common function. The organs suitably mimic any natural human or mammalian organ. Exemplary organs include, by way of non-limiting example trachea, bronchus, esophagus, stomach, intestine, colon, gall bladder, uterus, fallopian tube, ureter, bladder, urethra, lymph vessel, and the like, including portions thereof.

In some embodiments, the engineered tissues and organs are implantable. In further embodiments, implantable tissues and organs are biocompatible, meaning that they pose limited risk of injury or toxicity to organisms that they contact. In some embodiments, implantation involves inserting or grafting a tissue or organ into a subject. In further embodiments, insertion and/or grafting is performed surgically. In other embodiments, implantation involves affixing a tissue or organ to a subject. The tissues and organs disclosed herein are suitably implanted for various durations. In some embodiments, the tissues and/or organs are suitably implanted, by way of non-limiting example, temporarily, semi-permanently, and permanently. In some embodiments, implanted tissues and/or organs are absorbed, incorporated, or dissolved over time. In other embodiments, implanted tissues and/or organs retain a distinct form for some period of time.

The engineered tissues and organs are suitable for implantation in any vertebrate subject in need thereof. In various embodiments, vertebrate subjects include, by way of non-limiting examples, human subjects, vertebrate veterinary subjects, and those classified as Mammalia (mammals), Aves (birds), Reptilia (reptiles), Amphibia (amphibians), Osteichthyes (bony fishes), Chondrichthyes (cartilaginous fishes), Agnatha (jawless fishes), etc.

In various embodiments, engineered tissues and organs are suitable for implantation in any vertebrate subject in need of, for example, wound repair, tissue repair, tissue augmentation, tissue replacement, and/or organ replacement. In some embodiments, the engineered tissues are used for wound repair or tissue repair. For example, an engineered sheet is used to temporarily or permanently repair human skin damaged by injury. In some embodiments, the engineered tissues are used for tissue augmentation. For example, an engineered sheet is used to temporarily or permanently patch or repair a defect in the muscle wall of a human bladder or stomach. In some embodiments, the engineered tissues are used for tissue replacement. For example, an engineered sheet is used to temporarily or permanently repair or replace the wall of a segment of human small intestine. In some embodiments, the engineered organs are used for organ replacement.

The engineered, implantable tissues and organs, in various embodiments, are any suitable shape. In some embodiments, the shape is selected to mimic a particular natural tissue or organ. The engineered tissue or organ is a sheet and is not a vascular tube.

In some embodiments, a layer comprising muscle cells or an overall engineered tissue or organ is substantially in the form of a sheet or a form that comprises a sheet. In further embodiments, a sheet is a substantially planar form with a range of suitable geometries including, by way of non-limiting example, planar square, rectangle, polygon, circle, oval, or irregular. A bioprinted sheet has a wide range of suitable dimensions. In some embodiments, the dimensions are selected to facilitate a specific use including, by way of non-limiting examples, wound repair, tissue repair, tissue augmentation, tissue replacement, and engineered organ construction. In further embodiments, the dimensions are selected to facilitate a specific use in a specific subject. For instance, in one embodiment, a sheet is bioprinted to repair a particular defect in a muscle-comprising tissue of a specific human subject.

The engineered, implantable tissues and organs, in various embodiments, are any suitable size. In some embodiments, the size of engineered tissues and organs, including those bioprinted, change over time. In further embodiments, a bioprinted tissue or organ shrinks or contracts after bioprinting due to, for example, cell migration, cell death, cell-adhesion-mediated contraction, or other forms of shrinkage. In other embodiments, a bioprinted tissue or organs grows or expands after bioprinting due to, for example, cell migration, cell growth and proliferation, cell maturation, or other forms of expansion.

In some embodiments, a bioprinted sheet is at least 150 µm thick at the time of bioprinting. In various embodiments, a bioprinted sheet is about 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 µm or more thick, including increments therein. In further various embodiments, a bioprinted sheet is characterized by having a length, width, or both, of about 50, 100, 150, 200, 250, 300, 350, 400, 450, 500, 550, 600, 650, 700, 750, 800, 850, 900, 950, 1000 µm or more, including increments therein. In other various embodiments, a bioprinted sheet is characterized by having a length, width, or both, of about 1, 2, 3, 4, 5, 6, 7, 8, 9, 10, 11, 12, 13, 14, 15, 20, 25, 30, 35, 40, 45, 50, 55, 60, 65, 70, 75, 80, 85, 90, 95, 100 mm or more, including increments therein. In other various embodiments, a bioprinted sheet is characterized by having a length, width, or both, of about 1, 2, 3, 4, 5, 6, 7, 8, 9, 10, 11, 12, 13, 14, 15, 20, 25, 30, 35, 40, 45, 50, 55, 60, 65, 70, 75, 80, 85, 90, 95, 100 cm or more, including increments therein. *See,* e.g., **Example 6** (and **Fig. 1**), **Example 7** (and **Fig. 2**), **Example 9** (and **Figs. 3a** and **3b**), **Example 10** (and **Figs. 4a** and **4b**).

In some embodiments, a layer comprising muscle cells or an overall engineered tissue or organ is substantially in the form of a tube (not forming part of the invention) or a form that comprises a tube (not forming part of the invention). In further embodiments, a tube is a substantially a rolled sheet or a hollow cylinder (not forming part of the invention). *See,* e.g., **Example 13** (and **Fig. 6**).

In some embodiments, a layer comprising muscle cells or an overall engineered tissue or organ is substantially in the form of a sac (not forming part of the invention) or a form that comprises a sac (not forming part of the invention). In further embodiments, a sac is a substantially a rolled sheet or a hollow cylinder (not forming part of the invention) with at least one closed end (e.g., a pouch, cup, hollow, balloon, etc.). In some embodiments, a sac is an expandable structure intended for containment of ingested material, a fetus and related fluids, bodily fluids, or bodily wastes, and has at least one opening for input and at least one opening for output.

In some embodiments, an implantable tissue or organ is used for scientific and/or medical research. Suitable scientific and/or medical research includes both *in vivo* and *in vitro* research. In further embodiments, the engineered, tissues and/or organs described herein, are for *in vitro* research uses including, by way of non-limiting examples, disease modeling, drug discovery, and drug screening.

### Cells

Disclosed herein, in some embodiments, are engineered, implantable tissues and organs comprising one or more types of cells. The engineered tissues and organs include at least one layer comprising muscle cells. Therefore, in some embodiments, the cells include muscle cells (e.g., smooth muscle cells, skeletal muscle cells, cardiac muscle cells). In further embodiments, the layer comprising muscle cells also includes additional cells types such as those disclosed herein (e.g., fibroblasts, endothelial cells, etc.). In some embodiments, the engineered tissues and organs include cells other than muscle cells dispensed on at least one surface of a layer comprising muscle cells. In further embodiments, the cells dispensed on at least one surface of a layer comprising muscle cells include, by way of non-limiting examples, endothelial cells, nerve cells, pericytes, fibroblasts, tissue-specific epithelial cells, chondrocytes, skeletal muscle cells, cardiomyocytes, bone-derived cells, soft tissue-derived cells, mesothelial cells, tissue-specific stromal cells, stem cells, progenitor cells, and combinations thereof.

In some embodiments, any vertebrate cell is suitable for inclusion in the engineered, implantable tissues and organs. In further embodiments, the cells are, by way of non-limiting examples, contractile or muscle cells (e.g., skeletal muscle cells, cardiomyocytes, smooth muscle cells, and myoblasts), connective tissue cells (e.g., bone cells, cartilage cells, fibroblasts, and cells differentiating into bone forming cells, chondrocytes, or lymph tissues), bone marrow cells, endothelial cells, skin cells, epithelial cells, breast cells, vascular cells, blood cells, lymph cells, neural cells, Schwann cells, gastrointestinal cells, liver cells, pancreatic cells, lung cells, tracheal cells, corneal cells, genitourinary cells, kidney cells, reproductive cells, adipose cells, parenchymal cells, pericytes, mesothelial cells, stromal cells, undifferentiated cells (e.g., embryonic cells, stem cells, and progenitor cells), endoderm-derived cells, mesoderm-derived cells, ectoderm-derived cells, and combinations thereof.

In one embodiment, the cells are smooth muscle cells. In another embodiment, the cells are smooth muscle cells combined with at least one additional cell type. In some embodiments, the other cell type is fibroblasts. In some embodiments, the fibroblasts provide structural and biological support for the engineered tissue. In yet another embodiment, the other cell type is endothelial cells. In some embodiments, the endothelial cells facilitate vascularization and/or microvascularization of the engineered tissue. In still another embodiment, the cells are smooth muscle cells, fibroblasts, and endothelial cells. In embodiments including more than one cell type, the cell types are present in many suitable ratios, examples of which are described herein.

In some embodiments, the cells are adult, differentiated cells. In further embodiments, "differentiated cells" are cells with a tissue-specific phenotype consistent with, for example, a muscle cell, a fibroblast, or an endothelial cell at the time of isolation, wherein tissue-specific phenotype (or the potential to display the phenotype) is maintained from the time of isolation to the time of use. In other embodiments, the cells are adult, non-differentiated cells. In further embodiments, "non-differentiated cells" are cells that do not have, or have lost, the definitive tissue-specific traits of for example, muscle cells, fibroblasts, or endothelial cells. In some embodiments, non-differentiated cells include stem cells. In further embodiments, "stem cells" are cells that exhibit potency and self-renewal. Stem cells include, but are not limited to, totipotent cells, pluripotent cells, multipotent cells, oligopotent cells, unipotent cells, and progenitor cells. In various embodiments, stem cells are embryonic stem cells, adult stem cells, amniotic stem cells, and induced pluripotent stem cells. In other embodiments, the cells are a mixture of adult, differentiated cells and adult, non-differentiated cells.

In some embodiments, the smooth muscle cells are human smooth muscle cells. In some embodiments, suitable smooth muscle cells originated from tissue including, by way of non-limiting example, blood vessel, lymphatic vessel, tissue of the digestive tract, tissue of the genitourinary tract, adipose tissue, tissue of the respiratory tract, tissue of the reproductive system, bone marrow, and umbilical tissue. In some embodiments, additional (non-smooth-muscle) cellular components originated from the target tissue of interest. In other embodiments, additional (non-smooth-muscle) cellular components originated from a tissue other than the target tissue of interest. In further embodiments, some or all of the cells are cultured from the stromal vascular fraction of mammalian lipoaspirate. *See* **Example 1.**

In various embodiments, the cell types and/or source of the cells are selected, configured, treated, or modulated based on a specific goal or objective. In some embodiments, one or more specific cell types are derived from two or more distinct human donors. In some embodiments, one or more specific cell types are derived from a particular vertebrate subject. In further embodiments, one or more specific cell types are derived from a particular mammalian subject. In still further embodiments, one or more specific cell types are derived from a particular human subject.

### Methods of culturing cells

The cell types used in the engineered tissues of the invention are suitably cultured in any manner known in the art. Methods of cell and tissue culturing are known in the art, and are described, for example, in Freshney, R., Culture of Animal Cells: A Manual of Basic Techniques, Wiley (1987). General mammalian cell culture techniques, cell lines, and cell culture systems suitably used in conjunction with the present invention are also described in Doyle, A., Griffiths, J. B., Newell, D. G., (eds.) Cell and Tissue Culture: Laboratory Procedures, Wiley (1998).

Appropriate growth conditions for mammalian cells in culture are well known in the art. *See,* e.g., **Example 1**. Cell culture media generally include essential nutrients and, optionally, additional elements such as growth factors, salts, minerals, vitamins, etc., selected according to the cell type(s) being cultured. Particular ingredients are optionally selected to enhance cell growth, differentiation, secretion of specific proteins, etc. In general, standard growth media include Dulbecco's Modified Eagle Medium (DMEM), low glucose with 110 mg/L pyruvate and glutamine, supplemented with 1-20% fetal bovine serum (FBS), calf serum, or human serum and 100 U/mL penicillin, 0.1 mg/mL streptomycin are appropriate as are various other standard media well known to those in the art. Preferably cells are cultured under sterile conditions in an atmosphere of 1-21% O₂ and preferably 3-5% CO₂, at a temperature at or near the body temperature of the animal of origin of the cell. For example, human cells are preferably cultured at approximately 37°C.

The cells are optionally cultured with cellular differentiation agents to induce differentiation of the cell along the desired line. For instance, cells are optionally cultured with growth factors, cytokines, etc. In some embodiments, the term "growth factor" refers to a protein, a polypeptide, or a complex of polypeptides, including cytokines, that are produced by a cell and affect itself and/or a variety of other neighboring or distant cells. Typically growth factors affect the growth and/or differentiation of specific types of cells, either developmentally or in response to a multitude of physiological or environmental stimuli. Some, but not all, growth factors are hormones. Exemplary growth factors are insulin, insulin-like growth factor (IGF), nerve growth factor (NGF), vascular endothelial growth factor (VEGF), keratinocyte growth factor (KGF), fibroblast growth factors (FGFs), including basic FGF (bFGF), platelet-derived growth factors (PDGFs), including PDGF-AA and PDGF-AB, hepatocyte growth factor (HGF), transforming growth factor alpha (TGF-α), transforming growth factor beta (TGF-β), including TGFβ1 and TGFβ3, epidermal growth factor (EGF), granulocyte-macrophage colony-stimulating factor (GM-CSF), granulocyte colony-stimulating factor (G-CSF), interleukin-6 (IL-6), IL-8, and the like. Growth factors are discussed in, among other places, Molecular Cell Biology, Scientific American Books, Darnell et al., eds., 1986; Principles of Tissue Engineering, 2d ed., Lanza et al., eds., Academic Press, 2000.

### Bio-ink and multicellular aggregates

Disclosed herein, in certain embodiments, are engineered, implantable tissues and/or organs comprising a plurality of layers, wherein at least one layer comprises muscle cells. In some embodiments, the plurality of layers and/or the engineered tissue or organ consist essentially of cellular material. In further embodiments, cells other than muscle cells were dispensed on at least one surface of the one or more layers. The plurality of layers are substantially free of pre-formed scaffold at the time of bioprinting and at the time of use.

The cells and/or layers are bioprinted by extruding bio-ink from a bioprinter. "Bio-ink" includes semi-solid, or solid compositions comprising a plurality of cells. In some embodiments, bio-ink comprises semi-solid cell solutions, cell suspensions, or cell concentrations. In further embodiments, a cell solution, suspension, or concentration comprises a liquid or semi-solid (e.g., viscous) carrier and a plurality of cells. In still further embodiments, the carrier is a suitable cell nutrient media, such as those described herein. In some embodiments, bio-ink comprises semi-solid or solid multicellular aggregates or multicellular bodies. In further embodiments, the bio-ink is produced by 1) mixing a plurality of cells or cell aggregates and a biocompatible liquid or gel in a pre-determined ratio to result in bio-ink, and 2) compacting the bio-ink to produce the bio-ink with a desired cell density and viscosity. In some embodiments, the compacting of the bio-ink is achieved by centrifugation, tangential flow filtration ("TFF"), or a combination thereof.

The compacting of the bio-ink results in a composition that is extrudable, allowing formation of multicellular aggregates or multicellular bodies. In some embodiments, "extrudable" means able to be shaped by forcing (e.g., under pressure) through a nozzle or orifice (e.g., one or more holes or tubes). In some embodiments, the compacting of the bio-ink results from growing the cells to a suitable density. The cell density necessary for the bio-ink will vary with the cells being used and the tissue or organ being produced. In some embodiments, the cells of the bio-ink are cohered and/or adhered. In some embodiments, "cohere," "cohered," and "cohesion" refer to cell-cell adhesion properties that bind cells, multicellular aggregates, multicellular bodies, and/or layers thereof. In further embodiments, the terms are used interchangeably with "fuse," "fused," and "fusion." In some embodiments, the bio-ink additionally comprises support material, cell culture medium (or supplements thereof), extracellular matrix (not being a pre-formed scaffold) (or components thereof), cell adhesion agents, cell death inhibitors, anti-apoptotic agents, anti-oxidants, extrusion compounds, and combinations thereof.

In various embodiments, the cells are any suitable cell. In further various embodiments, the cells are vertebrate cells, mammalian cells, human cells, or combinations thereof. In some embodiments, the type of cell used in a method disclosed herein depends on the type of construct or tissue being produced. In some embodiments, the bio-ink comprises one type of cell (also referred to as a "homogeneous" or "monotypic" bio-ink). In some embodiments, the bio-ink comprises more than one type of cell (also referred to as a "heterogeneous" or "polytypic" bio-ink).

In various embodiments, bio-ink comprises 30, 35, 40, 45, 50, 55, 60, 65, 70, 75, 80, 85, 90, 95, 96, 97, 98, 99, 99.5, 99.9, and 100% cellular material, including increments therein, at the bio-ink is prepared. In various embodiments, bio-ink comprises 30, 35, 40, 45, 50, 55, 60, 65, 70, 75, 80, 85, 90, 95, 96, 97, 98, 99, 99.5, 99.9, and 100% cellular material, including increments therein, at the bio-ink is used in bioprinting.

### Cell culture media

In some embodiments, the bio-ink comprises a cell culture medium. The cell culture medium is any suitable medium. In various embodiments, suitable cell culture media include, by way of non-limiting examples, Dulbecco's Phosphate Buffered Saline, Earle's Balanced Salts, Hanks' Balanced Salts, Tyrode's Salts, Alsever's Solution, Gey's Balanced Salt Solution, Kreb's-Henseleit Buffer Modified, Kreb's-Ringer Bicarbonate Buffer, Puck's Saline, Dulbecco's Modified Eagle's Medium, Dulbecco's Modified Eagle's Medium/Nutrient F-12 Ham, Nutrient Mixture F-10 Ham (Ham's F-10), Medium 199, Minimum Essential Medium Eagle, RPMI-1640 Medium, Ames' Media, BGJb Medium (Fitton-Jackson Modification), Click's Medium, CMRL-1066 Medium, Fischer's Medium, Glascow Minimum Essential Medium (GMEM), Iscove's Modified Dulbecco's Medium (IMDM), L-15 Medium (Leibovitz), McCoy's 5A Modified Medium, NCTC Medium, Swim's S-77 Medium, Waymouth Medium, William's Medium E, or combinations thereof. In some embodiments, the cell culture medium is modified or supplemented. In some embodiments, the cell culture medium further comprises albumin, selenium, transferrins, fetuins, sugars, amino acids, vitamins, growth factors, cytokines, hormones, antibiotics, lipids, lipid carriers, cyclodextrins, platelet-rich plasma, or a combination thereof.

### Extracellular matrix

In some embodiments, the bio-ink further comprises one or more components of an extracellular matrix (not being a pre-formed scaffold) or derivatives thereof. In some embodiments, "extracellular matrix" includes proteins that are produced by cells and transported out of the cells into the extracellular space, where they serve as a support to hold tissues together, to provide tensile strength, and/or to facilitate cell signaling. Examples, of extracellular matrix components include, but are not limited to, collagen, fibronectin, laminin, hyaluronates, elastin, and proteoglycans. For example, the multicellular aggregates, in some cases, contain various ECM proteins (e.g., gelatin, fibrinogen, fibrin, collagen, fibronectin, laminin, elastin, and/or proteoglycans). In some embodiments, ECM components or derivatives of ECM components are added to the cell paste used to form the multicellular aggregate. In further embodiments, ECM components or derivatives of ECM components added to the cell paste are purified from a human or animal source, or produced by recombinant methods known in the art. Alternatively, the ECM components or derivatives of ECM components are naturally secreted by the cells in the elongate cellular body, or the cells used to make the elongate cellular body are genetically manipulated by any suitable method known in the art to vary the expression level of one or more ECM components or derivatives of ECM components and/or one or more cell adhesion molecules or cell-substrate adhesion molecules (e.g., selectins, integrins, immunoglobulins, and adherins). In some embodiments, ECM components or derivatives of ECM components promote cohesion of the cells in the multicellular aggregates. For example, in some embodiments, gelatin and/or fibrinogen is suitably be added to the cell paste, which is used to form multicellular aggregates. In further embodiments, the fibrinogen is converted to fibrin by the addition of thrombin.

In some embodiments, the bio-ink further comprises an agent that encourages cell adhesion.

In some embodiments, the bio-ink further comprises an agent that inhibits cell death (e.g., necrosis, apoptosis, or autophagocytosis). In some embodiments, the bio-ink further comprises an anti-apoptotic agent. Agents that inhibit cell death include, but are not limited to, small molecules, antibodies, peptides, peptibodies, or combination thereof. In some embodiments, the agent that inhibits cell death is selected from: anti-TNF agents, agents that inhibit the activity of an interleukin, agents that inhibit the activity of an interferon, agents that inhibit the activity of an GCSF (granulocyte colony-stimulating factor), agents that inhibit the activity of a macrophage inflammatory protein, agents that inhibit the activity of TGF-B (transforming growth factor B), agents that inhibit the activity of an MMP (matrix metalloproteinase), agents that inhibit the activity of a caspase, agents that inhibit the activity of the MAPK/JNK signaling cascade, agents that inhibit the activity of a Src kinase, agents that inhibit the activity of a JAK (Janus kinase), or a combination thereof. In some embodiments, the bio-ink comprises an anti-oxidant.

### Extrusion compounds

In some embodiments, the bio-ink further comprises an extrusion compound (i.e., a compound that modifies the extrusion properties of the bio-ink). Examples of extrusion compounds include, but are not limited to gels, hydrogels, peptide hydrogels, amino acid-based gels, surfactant polyols (e.g., Pluronic F-127 or PF-127), thermo-responsive polymers, hyaluronates, alginates, extracellular matrix components (and derivatives thereof), collagens, other biocompatible natural or synthetic polymers, nanofibers, and self-assembling nanofibers.

Gels, sometimes referred to as jellies, have been defined in various ways. For example, the United States Pharmacopoeia defines gels as semisolid systems consisting of either suspensions made up of small inorganic particles or large organic molecules interpenetrated by a liquid. Gels include a single-phase or a two-phase system. A single-phase gel consists of organic macromolecules distributed uniformly throughout a liquid in such a manner that no apparent boundaries exist between the dispersed macromolecules and the liquid. Some single-phase gels are prepared from synthetic macromolecules (e.g., carbomer) or from natural gums (e.g., tragacanth). In some embodiments, single-phase gels are generally aqueous, but will also be made using alcohols and oils. Two-phase gels consist of a network of small discrete particles.

Gels, in some cases, are classified as being hydrophobic or hydrophilic. In certain embodiments, the base of a hydrophobic gel consists of a liquid paraffin with polyethylene or fatty oils gelled with colloidal silica, or aluminum or zinc soaps. In contrast, the base of hydrophobic gels usually consists of water, glycerol, or propylene glycol gelled with a suitable gelling agent (e.g., tragacanth, starch, cellulose derivatives, carboxyvinylpolymers, and magnesium-aluminum silicates). In certain embodiments, the rheology of the compositions or devices disclosed herein is pseudo plastic, plastic, thixotropic, or dilatant.

Suitable hydrogels include those derived from collagen, hyaluronate, fibrin, alginate, agarose, chitosan, and combinations thereof. In other embodiments, suitable hydrogels are synthetic polymers. In further embodiments, suitable hydrogels include those derived from poly(acrylic acid) and derivatives thereof, poly(ethylene oxide) and copolymers thereof, poly(vinyl alcohol), polyphosphazene, and combinations thereof. In various specific embodiments, the confinement material is selected from: hydrogel, NovoGel™, agarose, alginate, gelatin, Matrigel™, hyaluronan, poloxamer, peptide hydrogel, poly(isopropyl n-polyacrylamide), polyethylene glycol diacrylate (PEG-DA), hydroxyethyl methacrylate, polydimethylsiloxane, polyacrylamide, poly(lactic acid), silicon, silk, or combinations thereof.

In some embodiments, hydrogel-based extrusion compounds are thermoreversible gels (also known as thermo-responsive gels or thermogels). In some embodiments, a suitable thermoreversible hydrogel is not a liquid at room temperature. In specific embodiments, the gelation temperature (Tgel) of a suitable hydrogel is about 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, including increments therein. In certain embodiments, the Tgel of a suitable hydrogel is about 10°C to about 40°C. In further embodiments, the Tgel of a suitable hydrogel is about 20°C to about 30°C. In some embodiments, the bio-ink (e.g., comprising hydrogel, one or more cell types, and other additives, etc.) described herein is not a liquid at room temperature. In some embodiments, a suitable thermoreversible hydrogel is not a liquid at mammalian body temperature. In specific embodiments, the gelation temperature (Tgel) of a suitable hydrogel is about 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41°C, 41°C, 43°C, 44°C, 45°C, 46°C, 47°C, 48°C, 49°C, 50°C, 51°C, 52°C, including increments therein. In certain embodiments, the Tgel of a suitable hydrogel is about 22°C to about 52°C. In further embodiments, the Tgel of a suitable hydrogel is about 32°C to about 42°C. In some embodiments, the bio-ink (e.g., comprising hydrogel, one or more cell types, and other additives, etc.) described herein is not a liquid at mammalian body temperature. In specific embodiments, the gelation temperature (Tgel) of a bio-ink described herein is about 10°C, about 15°C, about 20°C, about 25°C, about 30°C, about 35°C, about 40°C, about 45°C, about 50°C, about 55°C, including increments therein. In a specific embodiment, the Tgel of a bio-ink described herein is about 10°C to about 15°C. In another specific embodiment, the Tgel of a bio-ink described herein is about 15°C to about 20°C. In another specific embodiment, the Tgel of a bio-ink described herein is about 20°C to about 25°C. In another specific embodiment, the Tgel of a bio-ink described herein is about 25°C to about 30°C. In another specific embodiment, the Tgel of a bio-ink described herein is about 30°C to about 35°C. In another specific embodiment, the Tgel of a bio-ink described herein is about 35°C to about 40°C. In another specific embodiment, the Tgel of a bio-ink described herein is about 40°C to about 45°C. In another specific embodiment, the Tgel of a bio-ink described herein is about 45°C to about 50°C.

Polymers composed of polyoxypropylene and polyoxyethylene form thermoreversible gels when incorporated into aqueous solutions. These polymers have the ability to change from the liquid state to the gel state at temperatures maintainable in a bioprinter apparatus. The liquid state-to-gel state phase transition is dependent on the polymer concentration and the ingredients in the solution.

Poloxamer 407 (Pluronic F-127 or PF-127) is a nonionic surfactant composed of polyoxyethylene-polyoxypropylene copolymers. Other poloxamers include 188 (F-68 grade), 237 (F-87 grade), 338 (F-108 grade). Aqueous solutions of poloxamers are stable in the presence of acids, alkalis, and metal ions. PF-127 is a commercially available polyoxyethylene-polyoxypropylene triblock copolymer of general formula E106 P70 E106, with an average molar mass of 13,000. In some embodiments, the polymer is further purified by suitable methods that will enhance gelation properties of the polymer. It contains approximately 70% ethylene oxide, which accounts for its hydrophilicity. It is one of the series of poloxamer ABA block copolymers. PF-127 has good solubilizing capacity, low toxicity and is, therefore, considered a suitable extrusion compound.

In some embodiments, the viscosity of the hydrogels and bio-inks presented herein is measured by any means described. For example, in some embodiments, an LVDV-II+CP Cone Plate Viscometer and a Cone Spindle CPE-40 is used to calculate the viscosity of the hydrogels and bio-inks. In other embodiments, a Brookfield (spindle and cup) viscometer is used to calculate the viscosity of the hydrogels and bio-inks. In some embodiments, the viscosity ranges referred to herein are measured at room temperature. In other embodiments, the viscosity ranges referred to herein are measured at body temperature (e.g., at the average body temperature of a healthy human).

In further embodiments, the hydrogels and/or bio-inks are characterized by having a viscosity of between about 500 and 1,000,000 centipoise, between about 750 and 1,000,000 centipoise; between about 1000 and 1,000,000 centipoise; between about 1000 and 400,000 centipoise; between about 2000 and 100,000 centipoise; between about 3000 and 50,000 centipoise; between about 4000 and 25,000 centipoise; between about 5000 and 20,000 centipoise; or between about 6000 and 15,000 centipoise.

In some embodiments, the bio-ink comprises cells and extrusion compounds suitable for continuous bioprinting. In specific embodiments, the bio-ink has a viscosity of about 1500 mPa·s. Ion some embodiments, a mixture of Pluronic F-127 and cellular material is suitable for continuous bioprinting. In further embodiment, such a bio-ink is prepared by dissolving Pluronic F-127 powder by continuous mixing in cold (4 °C) phosphate buffered saline (PBS) over 48 hours to 30% (w/v). Pluronic F-127 is also suitably dissolved in water. Cells are optionally cultivated and expanded using standard sterile cell culture techniques. In some embodiments, the cells are pelleted at 200g for example, and re-suspended in the 30% Pluronic F-127. In further embodiments, the cells are aspirated into a reservoir affixed to a bioprinter and allowed to solidify at a gelation temperature from about 10 to about 25°C. Gelation of the bio-ink prior to bioprinting is optional. The bio-ink, including bio-ink comprising Pluronic F-127 is optionally dispensed as a liquid.

In various embodiments, the concentration of Pluronic F-127 is any value with suitable viscosity and/or cytotoxicity properties. In some embodiments, a suitable concentration of Pluronic F-127 is able to support weight while retaining its shape when bioprinted. In some embodiments, the concentration of Pluronic F-127 is about 10%, about 15%, about 20%, about 25%, about 30%, about 35%, about 40%, about 45%, or about 50%. In some embodiments, the concentration of Pluronic F-127 is between about 30% and about 40%, or between about 30% and about 35%.

In some embodiments, the non-cellular components of the bio-ink (e.g., extrusion compounds, etc.) are removed prior to use. In further embodiments, the non-cellular components are, for example, hydrogels, peptide hydrogels, amino acid-based gels, surfactant polyols, thermo-responsive polymers, hyaluronates, alginates, collagens, or other biocompatible natural or synthetic polymers. In still further embodiments, the non-cellular components are removed by physical, chemical, or enzymatic means. In some embodiments, a proportion of the non-cellular components remain associated with the cellular components at the time of use.

In some embodiments, the cells are pre-treated to increase cellular interaction. For example, cells are optionally incubated inside a centrifuge tube after centrifugation in order to enhance cell-cell interactions prior to shaping the bio-ink. By way of further example, cells are optionally exposed to molecules or reagents that facilitate cell-cell interactions, such as those that modulate ionic balance.

### Exemplary cell ratios

In some embodiments, the bio-ink utilized to build a tissue layer comprises multicellular bodies, which further comprise muscle cells (e.g., smooth muscle cells, skeletal muscle cells, and/or cardiac muscle cells) and one or more additional cell types. In further embodiments, the ratio of muscle cells to other cellular components is any suitable ratio. In still further embodiments, the ratio of muscle cells to other cellular components is about 90:10 to about 60:40. In a particular embodiment, the multicellular bodies comprise muscle cells and endothelial cells and the ratio of muscle cells to endothelial cells is about 85:15. In another particular embodiment, the multicellular bodies comprise muscle cells and endothelial cells and the ratio of muscle cells to endothelial cells is about 70:30.

In some embodiments, the bio-ink utilized to build a tissue layer comprises multicellular bodies which further comprise muscle cells and fibroblasts. In further embodiments, the ratio of muscle cells to fibroblasts is any suitable ratio. In still further embodiments, the ratio of muscle cells to fibroblasts is about 90:10 to about 60:40.

In some embodiments, the bio-ink utilized to build a tissue layer comprises multicellular bodies, which further comprise muscle cells, fibroblasts, and endothelial cells. In further embodiments, the ratio of muscle cells, fibroblasts, and endothelial cells is any suitable ratio. In still further embodiments, the ratio of muscle cells to fibroblasts and endothelial cells is about 70:25:5.

### Self-sorting of cells

In some embodiments, multicellular aggregates used to form the construct or tissue comprises all cell types to be included in the engineered tissue or organ (e.g., muscle cells and one or more additional cell types); in such an example, each cell type migrates to an appropriate position (e.g., during maturation) to form the engineered tissue or organ. In other embodiments, the multicellular aggregates used to form the structure comprises fewer than all the cell types to be included in the engineered tissue. In some embodiments, cells of each type are uniformly distributed within a multicellular aggregates, or region or layer of the tissue. In other embodiments, cells of each type localize to particular regions within a multicellular aggregate or layers or regions of the tissue.

For example, in the case of an engineered smooth muscle sheet comprising smooth muscle cells and endothelial cells in a suitable ratio (e.g., 85:15, 70:30, etc.), neighboring, bioprinted cohered polytypic cylindrical bio-ink units fuse. During maturation, endothelial cells localize to some extent to the periphery of the construct and collagen is formed. *See,* e.g., **Figs. 1****,** **2****,** **3a****,** and **4b****.** By way of further example, in the case of a bioprinted smooth muscle patch comprising smooth muscle cells, fibroblasts, and endothelial cells in a suitable ratio (e.g., 70:25:5, etc.), bioprinted polytypic cylindrical bio-ink units fuse and endothelial cells localize to some extent to the periphery of the construct. In some embodiments, localization of cell types within a construct mimics the layered structure of *in vivo* or *ex vivo* mammalian tissues. In some embodiments, the sorting or self-sorting of cells is accelerated, enhanced, or augmented by the application of one or more layers of cells. For example, in some embodiments, a construct bioprinted with polytypic bio-ink comprising smooth muscle cells and other cell types (such as endothelial cells and/or fibroblasts) is further subjected to application of a layer of a second cell type on one or more surfaces of the construct. In further embodiments, the result of applying a layer of a second cell type is augmentation of the spatial sorting of cells within the polytypic bio-ink.

### Pre-formed scaffold

According to the invention the engineered, implantable tissues and organs are free or substantially free of any pre-formed scaffold. In further embodiments, "scaffold" refers to synthetic scaffolds such as polymer scaffolds and porous hydrogels, non-synthetic scaffolds such as pre-formed extracellular matrix layers, dead cell layers, and decellularized tissues, and any other type of pre-formed scaffold that is integral to the physical structure of the engineered tissue and/or organ and not removed from the tissue and/or organ. In further embodiments, decellularized tissue scaffolds include decellularized native tissues or decellularized cellular material generated by cultured cells in any manner; for example, cell layers that are allowed to die or are decellularized, leaving behind the ECM they produced while living.

The engineered, implantable tissues and organs do not utilize any pre-formed scaffold, e.g., for the formation of the tissue, any layer of the tissue, or formation of the tissue's shape. As a non-limiting example, the engineered tissues of the present invention do not utilize any pre-formed, synthetic scaffolds such as polymer scaffolds, pre-formed extracellular matrix layers, or any other type of pre-formed scaffold. The engineered tissues and organs are substantially free of any pre-formed scaffolds at the time of bioprinting and at the time of use. In further embodiments, the tissues and organs contain a detectable, but trace or trivial amount of scaffold at the time of use, e.g., less than about 2.0% of the total composition. In still further embodiments, trace or trivial amounts of scaffold are insufficient to affect long-term behavior of the tissue or interfere with its primary biological function. In additional embodiments, scaffold components are removed post-printing, by physical, chemical, or enzymatic methods, yielding an engineered tissue that is free or substantially-free of scaffold components. In still further embodiments, the engineered, implantable tissues and organs contain biocompatible scaffold up to about 70% based on volume. In still further embodiments, the engineered, implantable tissues and organs contain biocompatible scaffold up to about 50% based on volume, at the time of use.

In some embodiments, the engineered, implantable tissues and organs free, or substantially free, of pre-formed scaffold disclosed herein are in stark contrast to those developed with certain other methods of tissue engineering in which a scaffolding material is formed in a first step, and then cells are seeded onto the scaffold in a second step. Subsequently the cells proliferate to fill and take the shape of the scaffold, for example. The methods of bioprinting described herein allow production of viable and useful tissues that are substantially free of pre-formed scaffold. In another aspect, the cells of the invention are, in some embodiments, held in a desired three-dimensional shape using a confinement material. The confinement material is distinct from a scaffold at least in the fact that the confinement material is temporary and/or removable from the cells and/or tissue.

### Layer comprising muscle cells

Disclosed herein, in certain embodiments, are engineered, implantable tissues and organs comprising a plurality of layers, wherein at least one layer of the engineered tissue or organ comprises muscle cells. In some embodiments, the engineered, implantable tissues and organs comprise at least one layer of muscle. A suitable layer comprising muscle cells and/or muscle includes cellular material. In various embodiments, a suitable layer comprising muscle cells has a composition of about 25, 30, 35, 40, 45, 50, 55, 60, 65, 70, 75, 80, 85, 90, 95, 96, 97, 98, 99, 99.5, 99.9, and 100% cellular material, including increments therein, at the time of construction. In other various embodiments, a suitable layer comprising muscle cells has a composition of about 25, 30, 35, 40, 45, 50, 55, 60, 65, 70, 75, 80, 85, 90, 95, 96, 97, 98, 99, 99.5, 99.9, and 100% cellular material, including increments therein, at the time of use. In some embodiments, the layer or layers comprising muscle cells comprise fused cellular elements in a three-dimensional geometry. In further embodiments, the layer or layers comprising muscle cells were bioprinted.

In some embodiments, a layer comprising muscle cells includes smooth muscle. In some embodiments, a layer comprising muscle cells includes skeletal muscle. In some embodiments, a layer comprising muscle cells includes cardiac muscle. In some embodiments, the layer or layers comprising muscle cells include any type of mammalian cell (in addition to muscle cells), such as those described herein. In various further embodiments, the layers include 1, 2, 3, 4, 5, 6, 7, 8, 9, 10, 11, 12, 13, 14, 15, 16, 17, 18, 19, 20 or more additional cell types. In some embodiments, the engineered tissues and organs include one or more cell types derived from one or more specific human subjects. In various embodiments, the engineered tissues and organs include cell types derived from 2, 3, 4, 5, 6, 7, 8, 9, 10, or more specific human subjects. In other embodiments, one or more specific cell types are derived from a particular vertebrate subject. In further embodiments, one or more specific cell types are derived from a particular mammalian subject. In still further embodiments, one or more specific cell types are derived from a particular human subject.

In some embodiments, a layer of smooth muscle includes smooth muscle cells and endothelial cells. **Example 3** demonstrates fabrication of cylindrical bio-ink consisting of human aortic smooth muscle cells and human aortic endothelial cells while **Example 5** demonstrates fabrication of bio-ink consisting of smooth muscle cells and endothelial cells cultured from the stromal vascular fraction of human lipoaspirate. **Example 6** demonstrates bioprinting and fusion of such cylinders to form smooth muscle patches. In other embodiments, a layer of smooth muscle includes smooth muscle cells and fibroblasts. In yet other embodiments, a layer of smooth muscle includes smooth muscle cells, endothelial cells, and fibroblasts. **Example 4** demonstrates fabrication of polytypic bio-ink consisting of human aortic smooth muscle cells, human dermal fibroblasts, and human aortic endothelial cells. In some embodiments, the cells of a layer of smooth muscle are "cohered" or "adhered" to one another. In further embodiments, cohesion or adhesion refers to cell-cell adhesion properties that bind cells, multicellular aggregates, multicellular bodies, and/or layers thereof.

The engineered, implantable tissues and organs comprising a plurality of layers, include any suitable number of layers. In various embodiments, the engineered tissues and organs include 2, 3, 4, 5, 6, 7, 8, 9, 10, 11, 12, 13, 14, 15 or more layers. In some embodiments, a layer is bioprinted and has an orientation defined by the placement, pattern, or orientation of multicellular bodies (e.g., elongate, cylindrical, or ribbon-like bodies). In further embodiments, an engineered tissue or organ includes a plurality of layer and each layer is characterized by having a particular orientation relative to one or more other layers. In various embodiments, one or more layers has an orientation that includes rotation relative to an adjacent layer, wherein the rotation is about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, or 180 degrees, or increments therein. In other embodiments, all layers are oriented substantially similarly.

A suitable layer is characterized by having any suitable thickness. In various embodiments, a suitable layer has a thickness of about 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 600, 610, 620, 630, 640, 650, 660, 670, 680, 690, 700, 750, 800, 850, 900, 950, 1000 µm or more, including increments therein, at the time of construction. In other various embodiments, a suitable layer has a thickness of about 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 600, 610, 620, 630, 640, 650, 660, 670, 680, 690, 700, 750, 800, 850, 900, 950, 1000 µm or more, including increments therein, at the time of use.

A suitable layer comprising muscle cells is characterized by having any suitable thickness. In various embodiments, a suitable layer comprising muscle cells has a thickness of about 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 600, 610, 620, 630, 640, 650, 660, 670, 680, 690, 700, 750, 800, 850, 900, 950, 1000 µm or more, including increments therein, at the time of construction. In other various embodiments, a suitable layer comprising muscle cells has a thickness of about 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 600, 610, 620, 630, 640, 650, 660, 670, 680, 690, 700, 750, 800, 850, 900, 950, 1000 µm or more, including increments therein, at the time of use.

In some embodiments, a layer comprising muscle cells is substantially in the form of a sheet or a form that comprises a sheet. In further embodiments, a bioprinted sheet of muscle is used to construct an engineered tissue or organ. In still further embodiments, a bioprinted sheet of muscle is used to surgically construct all or part of a muscle wall. In still further embodiments, a bioprinted sheet of muscle is used to surgically construct all or part of a gastrointestinal wall, a urologic wall, or an airway wall. In still further embodiments, a bioprinted sheet of muscle is used to surgically construct all or part of a bladder, a stomach, an intestine, an esophagus, a urethra, a uterus, a ureter, or a portion thereof. In still further embodiments, a bioprinted sheet of muscle is used to surgically construct all or part of a bladder wall, a stomach wall, an intestinal wall, an esophageal wall, a urethral wall, a uterine wall, a ureter wall, or a portion thereof.

In some embodiments, a layer comprising muscle cells is substantially in the form of a tube or a form that comprises a tube.

In some embodiments, a layer comprising muscle cells is substantially in the form of a sac or a form that comprises a sac.

### Cells other than muscle cells

The engineered, implantable tissues and organs disclosed herein include at least one layer comprising muscle cells. In further embodiments, the engineered, implantable tissues and organs disclosed herein include at least one layer comprising muscle and/or muscle cells. In still further embodiments, the engineered, implantable tissues and organs disclosed herein include at least one layer comprising smooth muscle and/or smooth muscle cells. In still further embodiments, the engineered, implantable tissues and organs disclosed herein include at least one layer comprising skeletal muscle and/or skeletal muscle cells. In still further embodiments, the engineered, implantable tissues and organs disclosed herein include at least one layer comprising cardiac muscle and/or cardiac muscle cells. In further embodiments, the engineered, implantable tissues and organs include cells other than muscle cells. In some embodiments, the cells other than muscle cells are incorporated into a layer comprising muscle cells. In various further embodiments, 1, 2, 3, 4, 5, 6, 7, 8, 9, 10, 11, 12, 13, 14, 15, or more types of cells are incorporated into a layer comprising muscle cells. In some embodiments, the cells other than muscle cells are dispensed on at least one surface of a layer comprising muscle cells. In various further embodiments, 1, 2, 3, 4, 5, 6, 7, 8, 9, 10, 11, 12, 13, 14, 15, or more types of cells are dispensed onto a layer comprising muscle cells. In still further various embodiments, cells other than muscle cells are dispensed onto 1, 2, 3, 4, or more surfaces of a layer comprising muscle cells.

In some embodiments, the cells dispensed on at least one surface of a layer comprising muscle cells include any type of mammalian cell, such as those described herein. In some embodiments, the dispensed cells include one or more cell types derived from one or more specific human subjects. In various embodiments, the dispensed cells include cell types derived from 2, 3, 4, 5, 6, 7, 8, 9, 10, or more specific human subjects. In other embodiments, one or more specific cell types are derived from a particular vertebrate subject. In further embodiments, one or more specific cell types are derived from a particular mammalian subject. In still further embodiments, one or more specific cell types are derived from a particular human subject.

In some embodiments, cells other than muscle cells are dispensed onto one or more surfaces of the muscle as a layer of cells. In further embodiments, a dispensed layer of cells comprises a monolayer of cells. In further embodiments, the monolayer is confluent. In other embodiments, monolayer is not confluent. In some embodiments, cells other than muscle cells are dispensed onto one or more surfaces of the muscle as one or more sheets of cells. In various embodiments, a sheet of cells is about 1, 2, 3, 4, 5, 6, 7, 8, 9, 10, 20, 30, 40, 50, 60, 70, 80, 90, 100 or more cells thick, including increments therein. In other various embodiments, a sheet of cells is about 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 µm or more thick, including increments therein. In some embodiments, cells other than muscle cells are dispensed onto one or more surfaces of the muscle as fused aggregates of cells. In further embodiments, prior to fusion, the aggregates of cells have, by way of non-limiting examples, substantially spherical, elongate, substantially cylindrical and ribbon-like shape. In various embodiments, fused aggregates of cells form a layer about 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 µm or more thick, including increments therein.

In some embodiments, the engineered tissues and organs include a second non-muscle cell type dispensed on one or more surfaces of a layer comprising muscle. **Example 7** demonstrates construction of a smooth muscle patch by bioprinting human vascular smooth muscle cell aggregates (e.g., cylinders) followed by bioprinting a layer of endothelial cells to the top surface of the SMC construct. **Example 8** demonstrates construction of smooth muscle patches by bioprinting human aortic smooth muscle cell aggregates (e.g., cylinders) followed by application of a layer of a second cell type to the top surface, achieved by deposition of specifically positioned droplets of an endothelial cell suspension onto the SMC construct. In some embodiments, the engineered tissues and organs include a third cell type, such as fibroblasts, dispensed on one or more surfaces of a layer of smooth muscle.

**Example 9** demonstrates construction of smooth muscle patches by bioprinting human aortic smooth muscle cell aggregates (e.g., cylinders) directly onto a layer comprised of a second cell type (e.g., fibroblasts), followed by application of a layer of a third cell type (e.g., endothelial cells) to the top surface. The top cell layer is applied by deposition of specifically positioned droplets of cell suspension onto the smooth muscle layer. The procedures of **Example 9** result in a tissue comprising cohered smooth muscle cells, a layer of fibroblasts on one surface of the smooth muscle cells, and a layer of endothelial cells on an opposing surface of the smooth muscle cells.

Cells other than muscle cells are dispensed into and/or onto one or more layers comprising muscle cells via any suitable technique. Suitable deposition techniques include those capable of delivering a somewhat controlled quantity or volume of cells without substantially damaging them. In various embodiments, suitable deposition techniques include, by way of non-limiting examples, spraying, ink-jetting, painting, dip coating, grafting, seeding, injecting, layering, bioprinting, and combinations thereof.

Cells other than muscle cells are dispensed on one or more layers comprising muscle cells at any suitable time in the fabrication process. In some embodiments, the cells are dispensed at substantially the same time as the muscle was fabricated or constructed (e.g., simultaneously, immediately thereafter, etc.). In other embodiments, the cells are dispensed following fabrication or construction of the muscle. In various further embodiments, the cells are dispensed 1, 2, 3, 4, 5, 6, 7, 8, 9, 10, 20, 30, 40, 50, 60, 70, 80, 90, or more minutes, including increments therein, following fabrication or construction of the muscle. In other various embodiments, the cells are dispensed 1, 2, 3, 4, 5, 6, 7, 8, 9, 10, 12, 24, 48, or more hours, including increments therein, following fabrication or construction of the muscle. In yet other various embodiments, the cells are dispensed 1, 2, 3, 4, 5, 6, 7, 8, 9, 10, or more days, including increments therein, following fabrication or construction of the muscle. In some embodiments, the cells are dispensed during maturation of one or more layers comprising muscle cells.

### Methods

Disclosed herein, in some embodiments, are methods of making implantable tissues or organs comprising a muscle cell-containing layer. In further embodiments, the method comprises bioprinting bio-ink comprising muscle cells into a form and fusing the bio-ink into a cohesive cellular structure. The implantable tissue or organ is substantially free of any pre-formed scaffold at the time of bioprinting and at the time of use. In various embodiments, the muscle cells are smooth muscle cells, skeletal muscle cells, and/ or cardiac muscle cells. The methods produce cell-comprising engineered tissues and organs substantially free of any pre-formed scaffold.

### Making bio-ink comprising muscle cells

In some embodiments, the methods involve making bio-ink comprising muscle cells. In some embodiments, the methods involve preparing cohered multicellular aggregates comprising muscle cells. In some embodiments, the methods involve preparing cohered multicellular aggregates further comprising other cell types. In further embodiments, the methods involve preparing multicellular aggregates further comprising endothelial cells. *See,* e.g., **Examples 3** and **5**. In some embodiments, the methods involve preparing cohered multicellular aggregates further comprising fibroblasts. *See,* e.g., **Example 4.**

There are various ways to make bio-ink comprising multicellular aggregates with the characteristics described herein. In some embodiments, a multicellular aggregate is fabricated from a cell paste containing a plurality of living cells or with a desired cell density and viscosity. In further embodiments, the cell paste is shaped into a desired shape and a multicellular body formed through maturation (e.g., incubation). In some embodiments, the multicellular aggregates are substantially cylindrical. In some embodiments, the multicellular aggregates are substantially spherical. In other embodiments, the engineered tissues are constructed from multicellular aggregates with a range of shapes. In a particular embodiment, an elongate multicellular body is produced by shaping a cell paste including a plurality of living cells into an elongate shape (e.g., a cylinder). In further embodiments, the cell paste is incubated in a controlled environment to allow the cells to adhere and/or cohere to one another to form the elongate multicellular body. In another particular embodiment, a multicellular body is produced by shaping a cell paste including a plurality of living cells in a device that holds the cell paste in a three-dimensional shape. In further embodiments, the cell paste is incubated in a controlled environment while it is held in the three dimensional shape for a sufficient time to produce a body that has sufficient cohesion to support itself on a flat surface.

In various embodiments, a cell paste is provided by: (A) mixing cells or cell aggregates (of one or more cell types) and a biocompatible gel or liquid, such as cell culture medium (e.g., in a pre-determined ratio) to result in a cell suspension, and (B) compacting the cellular suspension to produce a cell paste with a desired cell density and viscosity. In various embodiments, compacting is achieved by a number of methods, such as by concentrating a particular cell suspension that resulted from cell culture to achieve the desired cell concentration (density), viscosity, and consistency required for the cell paste. In a particular embodiment, a relatively dilute cell suspension from cell culture is centrifuged for a determined time to achieve a cell concentration in the pellet that allows shaping in a mold. Tangential flow filtration ("TFF") is another suitable method of concentrating or compacting the cells. In some embodiments, compounds are combined with the cell suspension to lend the extrusion properties required. Suitable compounds include, by way of non-limiting examples, surfactant polyols, collagens, hydrogels, Matrigel™, nanofibers, self-assembling nanofibers, gelatin, fibrinogen, etc.

In some embodiments, the cell paste is produced by mixing a plurality of living cells with a tissue culture medium, and compacting the living cells (e.g., by centrifugation). One or more ECM component (or derivative of an ECM component) is optionally included by, resuspending the cell pellet in one or more physiologically acceptable buffers containing the ECM component(s) (or derivative(s) of ECM component(s)) and the resulting cell suspension centrifuged again to form a cell paste.

In some embodiments, the cell density of the cell paste desired for further processing varies with cell types. In further embodiments, interactions between cells determine the properties of the cell paste, and different cell types will have a different relationship between cell density and cell-cell interaction. In still further embodiments, the cells are optionally pre-treated to increase cellular interactions before shaping the cell paste. For example, cells are optionally incubated inside a centrifuge tube after centrifugation in order to enhance cell-cell interactions prior to shaping the cell paste.

In various embodiments, many methods are used to shape the cell paste. For example, in a particular embodiment, the cell paste is manually molded or pressed (e.g., after concentration/compaction) to achieve a desired shape. By way of a further example, the cell paste is taken up (e.g., aspirated) into an instrument, such as a micropipette (e.g., a capillary pipette), that shapes the cell paste to conform to an interior surface of the instrument. The cross-sectional shape of the micropipette (e.g., capillary pipette) is alternatively circular, square, rectangular, triangular, or other non-circular cross-sectional shape. In some embodiments, the cell paste is shaped by depositing it into a preformed mold, such as a plastic mold, metal mold, or a gel mold. In some embodiments, centrifugal casting or continuous casting is used to shape the cell paste.

In some embodiments, substantially spherical multicellular aggregates, either alone or in combination with elongate cellular bodies, are also suitable to build the tissues and organs described herein. Spherical aggregates are suitably produced by a variety of methodologies, including self-assembly, the use of molds, and hanging drop methods. In further embodiments, a method to produce substantially spherical multicellular aggregates comprises the steps of 1) providing a cell paste containing a plurality of pre-selected cells or cell aggregates with a desired cell density and viscosity, 2) manipulating the cell paste into a cylindrical shape, 3) cutting cylinders into equal fragments, 4) letting the fragments round up overnight on a gyratory shaker, and 5) forming the substantially spherical multicellular aggregates through maturation.

In some embodiments, a partially adhered and/or cohered cell paste is transferred from the shaping device (e.g., capillary pipette) to a second shaping device (e.g., a mold) that allows nutrients and/or oxygen to be supplied to the cells while they are retained in the second shaping device for an additional maturation period. One example of a suitable shaping device that allows the cells to be supplied with nutrients and oxygen is a mold for producing a plurality of multicellular aggregates (e.g., substantially identical multicellular aggregates). By way of further example, such a mold includes a biocompatible substrate made of a material that is resistant to migration and ingrowth of cells into the substrate and resistant to adherence of cells to the substrate. In various embodiments, the substrate is suitably made of Teflon®, (PTFE), stainless steel, agarose, polyethylene glycol, glass, metal, plastic, or gel materials (e.g., hydrogel), and similar materials. In some embodiments, the mold is also suitably configured to allow supplying tissue culture media to the cell paste (e.g., by dispensing tissue culture media onto the top of the mold).

Thus, in embodiments where a second shaping device is used, the partially adhered and/or cohered cell paste is transferred from the first shaping device (e.g., a capillary pipette) to the second shaping device (e.g., a mold). In further embodiments, the partially adhered and/or cohered cell paste is transferred by the first shaping device (e.g., the capillary pipette) into the grooves of a mold. In still further embodiments, following a maturation period in which the mold is incubated along with the cell paste retained therein in a controlled environment to allow the cells in the cell paste to further adhere and/or cohere to one another to form the multicellular aggregate, the cohesion of the cells will be sufficiently strong to allow the resulting multicellular aggregate to be picked up with an implement (e.g., a capillary pipette). In still further embodiments, the capillary pipette is suitably be part of a printing head of a bioprinter or similar apparatus operable to automatically place the multicellular aggregate into a three-dimensional construct.

In some embodiments, the cross-sectional shape and size of the multicellular aggregates will substantially correspond to the cross-sectional shapes and sizes of the first shaping device and optionally the second shaping device used to make the multicellular aggregates, and the skilled artisan will be able to select suitable shaping devices having suitable cross-sectional shapes, cross-sectional areas, diameters, and lengths suitable for creating multicellular aggregates having the cross-sectional shapes, cross-sectional areas, diameters, and lengths discussed above.

In some embodiments, the bio-ink is formulated so that it is bioprintable using an automated, computer-aided, three-dimensional prototyping system capable of shaping and dispensing the bio-ink in a single step. In some embodiments, formulation of the bio-ink for single-step shaping and dispensing includes the addition of extrusion compounds.

### Bioprinting the bio-ink into a form

In some embodiments, the methods involve bioprinting bio-ink into a form. Bioprinting is a methodology described herein. Many three-dimensional forms are suitable and capable of production via bioprinting. The forms include sheets. In some embodiments, the form is bioprinted with dimensions suitable for replacing, partially replacing, or augmenting a native tissue or organ with an engineered, implantable tissue or organ. In further embodiments, the form is bioprinted with dimensions suitable for replacing, partially replacing, or augmenting a particular tissue or organ in a particular subject.

As described herein, in various embodiments, bio-ink comprises multicellular aggregates with many suitable shapes and sizes. In some embodiments, multicellular aggregates are elongate with any of several suitable cross-sectional shapes including, by way of non-limiting example, circular, oval, square, triangular, polygonal, and irregular. In further embodiments, multicellular aggregates are elongate and in the form of a cylinder. In some embodiments, elongate multicellular aggregates are of similar lengths and/or diameters. In other embodiments, elongate multicellular aggregates are of differing lengths and/or diameters. In some embodiments, multicellular aggregates are substantially spherical. In some embodiments, the engineered tissues include substantially spherical multicellular aggregates that are substantially similar in size. In other embodiments, the engineered tissues include substantially spherical multicellular aggregates that are of differing sizes. In some embodiments, engineered tissues of different shapes and sizes are formed by arranging multicellular aggregates of various shapes and sizes.

The cohered multicellular aggregates are placed onto a support. The support is any suitable biocompatible surface. In still further embodiments, suitable biocompatible surfaces include, by way of non-limiting examples, polymeric material, porous membranes, plastic, glass, metal, hydrogel, and combinations thereof. In some embodiments, the support is coated with a biocompatible substance including, by way of non-limiting examples, a hydrogel, a protein, a chemical, a peptide, antibodies, growth factors, or combinations thereof. In one embodiment, the support is coated with NovoGel™.

Once placement of the cohered multicellular aggregates is complete, in some embodiments, a tissue culture medium is poured over the top of the construct. In further embodiments, the tissue culture medium enters the spaces between the multicellular bodies to support the cells in the multicellular bodies.

The bioprinted form is a sheet. In further embodiments, a sheet is a substantially planar form with a range of suitable geometries including, by way of non-limiting example, planar square, rectangle, polygon, circle, oval, or irregular. A bioprinted sheet has a wide range of suitable dimensions. In some embodiments, the dimensions are selected to facilitate a specific use including, by way of non-limiting examples, wound repair, tissue repair, tissue augmentation, tissue replacement, and engineered organ construction. In further embodiments, the dimensions are selected to facilitate a specific use in a specific subject. For instance, in one embodiment, a sheet is bioprinted to repair a particular wound or defect in the muscle wall of an organ or tissue of a specific human subject. In some embodiments, a bioprinted sheet is at least 150 µm thick at the time of bioprinting. In various embodiments, a bioprinted sheet is about 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500 µm or more thick, including increments therein. In further various embodiments, a bioprinted sheet is characterized by having a length, width, or both, of about 50, 100, 150, 200, 250, 300, 350, 400, 450, 500, 550, 600, 650, 700, 750, 800, 850, 900, 950, 1000 µm or more, including increments therein. In other various embodiments, a bioprinted sheet is characterized by having a length, width, or both, of about 1, 2, 3, 4, 5, 6, 7, 8, 9, 10, 11, 12, 13, 14, 15, 20, 25, 30, 35, 40, 45, 50, 55, 60, 65, 70, 75, 80, 85, 90, 95, 100 mm or more, including increments therein. In other various embodiments, a bioprinted sheet is characterized by having a length, width, or both, of about 1, 2, 3, 4, 5, 6, 7, 8, 9, 10, 11, 12, 13, 14, 15, 20, 25, 30, 35, 40, 45, 50, 55, 60, 65, 70, 75, 80, 85, 90, 95, 100 cm or more, including increments therein. *See,* e.g., **Example 6** (and **Fig. 1**), **Example 7** (and **Fig. 2**), **Example 9** (and **Figs. 3a** and **3b**)**, Example 10** (and **Figs. 4a** and **4b**).

In some embodiments, the bioprinted form is a tube (not forming part of the invention). In further embodiments, a tube is a substantially a rolled sheet or a hollow cylinder (not forming part of the invention). *See,* e.g., **Example 13** (and **Fig. 6**).

In some embodiments, the bioprinted form is a sac (not forming part of the invention). In further embodiments, a sac is a substantially a rolled sheet or a hollow cylinder (not forming part of the invention) with at least one closed end (e.g., a pouch, cup, hollow, balloon, etc.).

### Fusion of the bio-ink

In some embodiments, the methods involve fusing bio-ink into a cohesive cellular structure. In further embodiments, fusion of the bio-ink comprising multicellular aggregates is facilitated by incubation. In further embodiments, the incubation allows the multicellular aggregates adhere and/or cohere to form a tissue or an organ. In some embodiments, the multicellular aggregates cohere to form a tissue in a cell culture environment (e.g., a Petri dish, cell culture flask, bioreactor, etc.). In further embodiments, the multicellular aggregates cohere to form a tissue in an environment with conditions suitable to facilitate growth of the cell types included in the multicellular aggregates. In one embodiment, the multicellular aggregates are incubated at about 37°C, in a humidified atmosphere containing about 5% CO₂, containing about 1%-21% O₂, in the presence of cell culture medium containing factors and/or ions to foster adherence and/or coherence.

The incubation, in various embodiments, has any suitable duration. In further various embodiments, the incubation has a duration of about 20, 30, 40, 50, 60, 70, 80, 90, 100, 110, 120, 130, 140, 150, 160, 170, 180, or more minutes, including increments therein. In further various embodiments, the incubation has a duration of about 1, 2, 3, 4, 5, 6, 7, 8, 9, 10, 11, 12, 13, 14, 15, 16, 17, 18, 19, 20, 21, 22, 23, 24, 36, 48, or more hours, including increments therein. In further various embodiments, the incubation has a duration of about 1, 2, 3, 4, 5, 6, 7, 8, 9, 10, 11, 12, 13, 14, 15, 16, 17, 18, 19, 20, or more days, including increments therein. Several factors influence the time required for multicellular aggregates to cohere to form a tissue including, by way of non-limiting examples, cell types, cell type ratios, culture conditions, and the presence of additives such as growth factors.

### Applying cells into or onto the bioprinted form

In some embodiments, the methods further involve applying cells into or onto the bioprinted form. A number of methods and techniques are suitable to apply the cells. In various further embodiments, the cells are, for example, bioprinted, sprayed, painted, dip coated, grafted, seeded, injected, layered or bioprinted into or onto the form. For example, in some embodiments, applying cells comprises coating one or more surfaces of a muscle construct with a suspension, sheet, monolayer, or fused aggregates of cells. In various embodiments, 1, 2, 3, 4, or more surfaces of the muscle construct are coated.

In some embodiments, applying cells comprises bioprinting an additional layer of fused multicellular aggregates. In other embodiments, applying a layer of cells comprises bioprinting, spraying, or ink-jetting a solution, suspension, or liquid concentrate of cells. In further embodiments, a suitable cell suspension comprises about 1 x 10⁴ to about 1 x 10⁶ cells/µL. In still further embodiments, a suitable cell suspension comprises about 1 x 10⁵ to about 1.5 x 10⁵ cells/µL. In further embodiments, applying cells comprises dispensing a suspension of cells directly onto one or more surfaces of a tissue construct as spatially-distributed droplets. In still further embodiments, applying cells comprises dispensing a suspension of cells directly onto one or more surfaces of a tissue construct as a spray. Layers of cells are, in various embodiments, applied at any suitable time in the construction process. In some embodiments, one or more layers of cells are applied on one or more external surfaces of the smooth muscle construct immediately after bioprinting (e.g., up to 10 min.). In other embodiments, one or more layers are applied after bioprinting (e.g., after 10 min.). In yet other embodiments, one or more layers are applied during maturation of the construct.

Any type of cell is suitable for application by bioprinting as cohered multicellular aggregates. Moreover, any type of cell is suitable for application by deposition as droplets of suspension, solution, or concentrate, or spraying as a suspension, solution, or concentrate. In some embodiments, fibroblasts are applied on one or more external surfaces of the smooth muscle construct. In other embodiments, endothelial cells are applied on one or more external surfaces of the smooth muscle construct. In further embodiments, a layer of endothelial cells is applied to one or more external surfaces of the smooth muscle construct and a layer of fibroblasts is applied to one or more distinct surfaces of the construct.

**Example 7** demonstrates smooth muscle constructs bioprinted with cohered smooth muscle cell aggregates, which were further coated with a second cell type consisting of an endothelial cell concentrate (e.g., 1-1.5x10⁵ cells/µl). The techniques of **Example 7** resulted in a smooth muscle construct comprised of SMC. *See,* e.g., **Fig. 2****.**

**Example 8** demonstrates smooth muscle constructs bioprinted with cohered human aortic smooth muscle cell aggregates. Further, a second cell type consisting of human aortic endothelial cells in suspension was dispensed from a bioprinter on top of the bioprinted smooth muscle cell layer as 2.5 µL droplets.

In some embodiments, the methods further comprise the step of culturing a layer of cells on a support. In such embodiments, applying cells, in some cases, comprises placing one or more surfaces of the smooth muscle construct in direct contact with a pre-existing layer of cells. In further embodiments, the construct is bioprinted directly onto a cultured layer of cells or a monolayer of cells. Any type of cultured cell layer on a biocompatible support is suitable. In some embodiments, multicellular aggregates are bioprinted onto a layer of endothelial cells. In other embodiments, multicellular aggregates are bioprinted onto a layer of fibroblasts. In further embodiments, the layer of cells adheres and/or coheres with the multicellular aggregates of the bioprinted construct.

**Example 9** demonstrates construction of the same constructs of **Example 8;** however, the constructs were bioprinted onto a support on which a confluent monolayer of human dermal fibroblasts had been pre-cultured. The techniques of **Example 9** resulted in a smooth muscle construct comprised of SMC with additional layers comprising both an endothelial layer and a fibroblast layer. *See,* e.g., **Figs. 3a** and **3b****.**

### Additional steps for increasing viability of the engineered tissue

In some embodiments, the method further comprises steps for increasing the viability of the engineered tissue or organ after bioprinting and before implantation. In further embodiments, these steps involve providing direct contact between the tissue or organ and a nutrient medium through a temporary or semi-permanent lattice of confinement material. In some embodiments, the tissue is constrained in a porous or gapped material. Direct access of at least some of the cells of the engineered tissue to nutrients increases the viability of the engineered tissue.

In further embodiments, the additional and optional steps for increasing the viability of the engineered tissue include: 1) optionally disposing base layer of confinement material prior to placing cohered multicellular aggregates; 2) optionally disposing a perimeter of confinement material; 3) bioprinting cells of the tissue within a defined geometry; and 4) disposing elongate bodies (e.g., cylinders, ribbons, etc.) of confinement material overlaying the nascent tissue in a pattern that introduces gaps in the confinement material, such as a lattice, mesh, or grid. *See,* e.g., **Example 12** and **Fig. 5****.**

Many confinement materials are suitable for use in the methods described herein. In some embodiments, hydrogels are exemplary confinement materials possessing one or more advantageous properties including: non-adherent, biocompatible, extrudable, bioprintable, non-cellular, of suitable strength, and not soluble in aqueous conditions. In some embodiments, suitable hydrogels are natural polymers. In one embodiment, the confinement material is comprised of NovoGel™. In further embodiments, suitable hydrogels include those derived from surfactant polyols such as Pluronic F-127, collagen, hyaluronate, fibrin, alginate, agarose, chitosan, derivatives or combinations thereof. In other embodiments, suitable hydrogels are synthetic polymers. In further embodiments, suitable hydrogels include those derived from poly(acrylic acid) and derivatives thereof, poly(ethylene oxide) and copolymers thereof, poly(vinyl alcohol), polyphosphazene, and combinations thereof. In various specific embodiments, the confinement material is selected from: hydrogel, NovoGel™, agarose, alginate, gelatin, Matrigel™, hyaluronan, poloxamer, peptide hydrogel, poly(isopropyl n-polyacrylamide), polyethylene glycol diacrylate (PEG-DA), hydroxyethyl methacrylate, polydimethylsiloxane, polyacrylamide, poly(lactic acid), silicon, silk, or combinations thereof.

In some embodiments, the gaps overlaying pattern are distributed uniformly or substantially uniformly around the surface of the tissue. In other embodiments, the gaps are distributed non-uniformly, whereby the cells of the tissue are exposed to nutrients non-uniformly. In non-uniform embodiments, the differential access to nutrients is exploited to influence one or more properties of the tissue. For instance, in some cases it is desirable to have cells on one surface of a bioprinted tissue proliferate faster than cells on another surface of the bioprinted tissue. In some embodiments, the exposure of various parts of the tissue to nutrients is optionally changed at various times to influence the development of the tissue toward a desired endpoint.

In some embodiments, the confinement material is removed at any suitable time, including but not limited to, immediately after bioprinting (e.g., within 10 minutes), after bioprinting (e.g., after 10 minutes), before the cells are substantially cohered to each other, after the cells are substantially cohered to each other, before the cells produce an extracellular matrix, after the cells produce an extracellular matrix, just prior to use, and the like. In various embodiments, confinement material is removed by any suitable method. For example, in some embodiments, the confinement material is excised, pulled off the cells, digested, or dissolved.

In some embodiments, the methods further comprise the step of subjecting the engineered tissue to shear force, caused by fluid flow, on one or more sides.

### Particular exemplary embodiments

In certain embodiments, disclosed herein are engineered tissues and organs comprising at a plurality of layer comprising muscle cells, wherein the engineered tissue or organ consists essentially of cellular material and is implantable in a vertebrate subject, and wherein the engineered tissue or organ is is a sheet and is not a blood vessel. In some embodiments, the tissue or organ is a sheet and is not a blood vessel. In some embodiments, the layer of muscle was formed by fusion of bioprinted aggregates of cells. The layer of muscle is substantially free of any pre-formed scaffold at the time of bioprinting and at the time of use. The layer of muscle was not shaped using a pre-formed scaffold. In some embodiments, the tissue or organ consists essentially of cellular material that generates an extracellular matrix following bioprinting. In some embodiments, the layer of muscle is smooth muscle and is at least 150 µm thick at the time of bioprinting. In further embodiments, the layer of smooth muscle is at least about 250 µm at the time of bioprinting. In further embodiments, the layer of smooth muscle is at least about 500 µm thick at the time of bioprinting. In some embodiments, the tissue or organ further comprises cells selected from the group consisting of: endothelial cells, nerve cells, pericytes, fibroblasts, tissue-specific epithelial cells, chondrocytes, skeletal muscle cells, cardiomyocytes, bone-derived cells, soft tissue-derived cells, mesothelial cells, tissue-specific stromal cells, stem cells, progenitor cells, and combinations thereof. In some embodiments, cells other than smooth muscle cells are dispensed on at least one surface of the layer of smooth muscle. In further embodiments, cells other than smooth muscle cells were bioprinted on at least one surface of the layer of smooth muscle. In still further embodiments, the cells are selected from the group consisting of: endothelial cells, nerve cells, pericytes, fibroblasts, tissue-specific epithelial cells, chondrocytes, skeletal muscle cells, cardiomyocytes, bone-derived cells, soft tissue-derived cells, mesothelial cells, tissue-specific stromal cells, stem cells, progenitor cells, and combinations thereof. In some embodiments, the cells other than smooth muscle cells were dispensed on the smooth muscle layer at substantially the same time as the smooth muscle layer was bioprinted. In some embodiments, the cells other than smooth muscle cells were dispensed on the smooth muscle layer following bioprinting of the smooth muscle layer. In some embodiments, the cells other than smooth muscle cells were dispensed on the smooth muscle layer during maturation of the smooth muscle layer. In some embodiments, the cells other than smooth muscle cells were dispensed on the smooth muscle layer following maturation of the smooth muscle layer. In some embodiments, the cells other than smooth muscle cells were dispensed on the smooth muscle layer within 24 hours of bioprinting the smooth muscle layer. In some embodiments, the cells other than smooth muscle cells were dispensed on the smooth muscle layer following 24 hours after bioprinting the smooth muscle layer. In some embodiments, cells other than smooth muscle cells were dispensed on the layer of smooth muscle as one or more layers of cells. In some embodiments, cells other than smooth muscle cells were dispensed on the layer of smooth muscle as a layer of cells less than about 100 µm thick. In other embodiments, cells other than smooth muscle cells were dispensed on the layer of smooth muscle as a layer of cells greater than about 100 µm thick and less than about 500 µm thick. In some embodiments, fibroblast cells were dispensed in or on the layer of smooth muscle. In some embodiments, endothelial cells were dispensed in or on the layer of smooth muscle. In further embodiments, the endothelial cells are tissue-specific. In some embodiments, the layer of smooth muscle is substantially planar. In further embodiments, the plane is at least 150 µm thick at the time of bioprinting. In still further embodiments, the tissue is a smooth muscle cell-comprising sheet or patch suitable for wound repair or tissue augmentation. In some embodiments, the layer of smooth muscle was bioprinted with dimensions suitable for replacing a native organ with the engineered implantable organ. In some embodiments, the layer of smooth muscle was bioprinted with dimensions suitable for partially replacing a native organ with the engineered implantable organ. In some embodiments, the layer of smooth muscle was bioprinted with dimensions suitable for augmenting a native organ with the engineered implantable organ. In some embodiments, the smooth muscle-comprising sheet was supported by a non-adherent hydrogel confinement material during bioprinting. In further embodiments, the non-adherent hydrogel confinement material remained associated with the smooth muscle-comprising sheet after bioprinting. In still further embodiments, the non-adherent hydrogel confinement material was dissociated from the smooth muscle-comprising sheet at some time point after bioprinting and before implantation *in vivo.* In further embodiments, non-adherent hydrogel confined the bioprinted cells to the suitable dimensions. In still further embodiments, the non-adherent hydrogel confinement material was configured to allow at least some of the bioprinted cells to contact a nutrient medium. In some embodiments, the cells comprise adult, differentiated cells. In other embodiments, the cells comprise adult, non-differentiated cells. In some embodiments, the smooth muscle cells are tissue-specific. In further embodiments, the smooth muscle cells are human aortic smooth muscle cells or human umbilical vein smooth muscle cells. In some embodiments, the smooth muscle cells are derived from human lipoaspirate. In some embodiments, the tissue or organ comprises additional non-smooth-muscle cell types derived from human lipoaspirate. In some embodiments, the cells are derived from a particular vertebrate subject. In some embodiments, the cells are selected to mimic a particular disease state. In some embodiments, the tissue or organ is selected from the group consisting of: urethra, urinary conduit, ureter, bladder, fallopian tube, uterus, trachea, bronchus, lymphatic vessel, esophagus, stomach, gallbladder, small intestine, large intestine, and colon.

In certain embodiments, disclosed herein is implantation of the engineered tissues and/or organs in a vertebrate subject, wherein the tissues and organs comprise at least one layer of smooth muscle, wherein the engineered tissue or organ consists essentially of cellular material, and wherein the engineered tissue or organ is not a blood vessel.

In certain embodiments, disclosed herein are methods for making an implantable tissue or organ comprising smooth muscle tissue, the method comprising: making bio-ink comprising smooth muscle cells; bioprinting the bio-ink into a form; and fusion of the bio-ink into a cohesive cellular structure, wherein the implantable tissue or organ a sheet and is not a blood vessel. In some embodiments, the implantable tissue or organ is substantially free of any pre-formed scaffold. In some embodiments, the smooth muscle cells are isolated from native smooth muscle tissues of a mammalian subject. In some embodiments, the smooth muscle cells are differentiated from progenitors. In some embodiments, the smooth muscle cells are generated from a tissue sample. In further embodiments, the tissue sample is lipoaspirate. In some embodiments, the form is matured for about 2 hours to about 10 days. In further embodiments, maturation occurs over a period of up to 4 weeks. The form is a sheet. In some embodiments, the bio-ink further comprises cells selected from the group consisting of: endothelial cells, nerve cells, pericytes, fibroblasts, tissue-specific epithelial cells, non-vascular smooth muscle cells, chondrocytes, skeletal muscle cells, cardiomyocytes, bone-derived cells, soft tissue-derived cells, mesothelial cells, tissue-specific stromal cells, stem cells, progenitor cells, and combinations thereof. In some embodiments, the method further comprises the step of bioprinting, spraying, painting, applying, dip coating, grafting, seeding, injecting, or layering cells into or onto the bioprinted form. In some embodiments, the method further comprises the step of bioprinting, spraying, painting, applying, dip coating, grafting, injecting, seeding, or layering cells into or onto the cohesive cellular structure. In some embodiments, the method further comprises the step of biomechanically or biochemically conditioning the bioprinted form to mature toward a targeted application.

In certain embodiments, disclosed herein are engineered tissues for use in making an implantable engineered organ, wherein said tissue comprises at least one layer of smooth muscle; wherein said at least one layer of smooth muscle comprises fused cellular elements in a three-dimensional geometry, and wherein the tissue is a sheet and is not a blood vessel. The at least one layer of smooth muscle was bioprinted. In further embodiments, the tissue is substantially free of any pre-formed scaffold. In some embodiments, the three-dimensional geometry was confined by a non-adherent material or mold. In some embodiments, the tissue further comprises cells selected from the group consisting of: endothelial cells, nerve cells, pericytes, fibroblasts, tissue-specific epithelial cells, non-vascular smooth muscle cells, chondrocytes, skeletal muscle cells, cardiomyocytes, bone-derived cells, soft tissue-derived cells, mesothelial cells, tissue-specific stromal cells, stem cells, progenitor cells, and combinations thereof. In some embodiments, cells other than smooth muscle cells are dispensed on at least one surface of the layer of smooth muscle. In further embodiments, cells other than smooth muscle cells are bioprinted on at least one surface of the layer of smooth muscle. In still further embodiments, the cells other than smooth muscle cells are selected from the group consisting of: endothelial cells, nerve cells, pericytes, fibroblasts, tissue-specific epithelial cells, non-vascular smooth muscle cells, chondrocytes, skeletal muscle cells, cardiomyocytes, bone-derived cells, soft tissue-derived cells, mesothelial cells, tissue-specific stromal cells, stem cells, progenitor cells, and combinations thereof. In some embodiments, the cellular layer is at least 150 µm thick at the time of bioprinting. In some embodiments, the tissue is affixed to a tissue culture-compatible surface. In some embodiments, the tissue is suitable for implantation in a vertebrate subject. In certain embodiments, disclosed herein are engineered tissue culture systems comprising a three-dimensional cell-based element and a temporary or removable confinement, wherein the confinement material allows for direct contact between the cells and a nutrient medium. The engineered, three-dimensional cell-based element was bioprinted. In further embodiments, the engineered, three-dimensional cell-based element is free of any pre-formed scaffold. In some embodiments, the confinement material has at least one of the following features: does not substantially adhere to the cells; is biocompatible; is extrudable; is non-cellular; is of sufficient strength to provide support for the cells; and is not soluble in aqueous conditions. In further embodiments, the confinement material is not plastic, is not glass, and is not ceramic. In some embodiments, the confinement material is a hydrogel. In further embodiments, the confinement material is NovoGel™. In further embodiments, the confinement material comprises one or more of: agarose, polyethylene glycol diacrylate (PEG-DA), hyaluronan, gelatin, poloxamer, hydroxyethyl methacrylate, peptide hydrogel, Matrigel™, polydimethylsiloxane, silicon, silk, polyacrylamide, poly lactic acid, a surfactant polyol, and alginate. In some embodiments, at least one of: the cells and/or the confinement material were extruded from a bioprinter. In further embodiments, there are gaps in the confinement material and wherein the nutrient medium is capable of contacting the cells through the gaps. In still further embodiments, the gaps were between about 100 µm and about 30 mm wide. In some embodiments, the gaps were distributed non-uniformly around the structure, whereby the cells of the tissue were exposed to nutrients non-uniformly. In some embodiments, wherein at least about 10% of the surface area of the tissue was exposed to gaps suitable for contact with a nutrient medium. In some embodiments, the confinement material was overlaid on the cells as at least one elongated element. In further embodiments, the elongated element of confinement material had a cross-sectional thickness between about 100 µm and about 1 mm. In some embodiments, there were gaps between the elongated elements of confinement material. In some embodiments, gaps were left between elongated elements when extruding the confinement material from a bioprinter. In other embodiments, at least some of the confinement material was removed from the system to provide gaps. In some embodiments, the elongated elements of confinement material were substantially parallel and the gaps were elongated. In some embodiments, the elongated elements of confinement material were arranged in a lattice. In some embodiments, the elongated elements of confinement material affix the structure to the supporting surface. In some embodiments, the system was suitable for shipping. In some embodiments, the bioprinted cells comprise at least one of: smooth muscle cells, endothelial cells, fibroblasts, and epithelial cells. In some embodiments, the nutrient medium comprised at least one of: oxygen (O₂), a carbon source, a nitrogen source, growth factors, salts, minerals, vitamins, serum, antibiotics, chemicals, proteins, nucleic acids, pharmaceutical compounds, and antibodies.

In certain embodiments, disclosed herein are engineered, living tissues comprising a three-dimensional cell-comprising element, held in place by a hydrogel, wherein the hydrogel was dispensed on said cell-comprising element as cylinders or ribbons with gaps between the cylinders or ribbons through which the cells access nutrients, and wherein the hydrogel is removable from the tissue.

In certain embodiments, disclosed herein are methods for increasing the viability of an engineered tissue comprising providing direct contact between the tissue and a nutrient medium through a temporary or semi-permanent lattice, wherein the tissue is free of any pre-formed scaffold. In some embodiments, the step of providing direct contact between the tissue and a nutrient medium through a temporary or semi-permanent lattice comprises constraining said tissue in a porous or gapped material. In further embodiments, the pores or gaps were between about 100 µm and about 30 mm wide. In further embodiments, the porous or gapped material was a hydrogel. In still further embodiments, the porous or gapped material was agarose. In some embodiments, viability of an engineered tissue is increased *ex vivo.* In some embodiments, viability of at least a portion of the cells comprising an engineered tissue is extended. In further embodiments, viability of the cells is extended by 1 day or more. In some embodiments, the at least one nutrient is selected from the group consisting of: a carbon source, a nitrogen source, growth factors, salts, minerals, vitamins, serum, antibiotics, proteins, nucleic acids, pharmaceutical compounds, ad antibodies. In some embodiments, at least one nutrient is oxygen (O₂). In further embodiments, the porous or gapped hydrogel confinement is designed to provide the bioprinted cells with differential exposure to nutrients on one or more surfaces.

In certain embodiments, disclosed herein are methods of making tissue culture systems comprising the steps of: establishing a three-dimensional cell-comprising element on a biocompatible substrate; and dispensing confinement material overlaying the three-dimensional cell-comprising element, wherein the overlaid confinement material allows at least some of the cells to contact a growth medium.

In certain embodiments, disclosed herein are methods of making tissue culture systems comprising the steps of: dispensing a perimeter of confinement material on a surface; dispensing cells within the perimeter; and dispensing confinement material overlaying the cells, wherein the overlaid confinement material allows at least some of the cells to contact a growth medium. In some embodiments, dispensing confinement material is accomplished by bioprinting. In some embodiments, the method comprises or further comprises culturing the system in a suitable medium to mature the bioprinted cellular construct.

### EXAMPLES

The following specific examples are to be construed as merely illustrative, and not limitative of the remainder of the disclosure in any way whatsoever.

### Example 1 - Cell culture

### Smooth muscle cells

Primary human aortic smooth muscle cells (HASMC; GIBCO/Invitrogen Corp., Carlsbad, CA) were maintained and expanded in low glucose dulbecco's modified eagle medium (DMEM; Invitrogen Corp., Carlsbad, CA) supplemented with 10% fetal bovine serum (FBS), 100 U/mL Penicillin, 0.1 mg/mL streptomycin, 0.25 µg/mL of amphotericin B, 0.01M of HEPES (all from Invitrogen Corp., Carlsbad, CA), 50 mg/L of proline, 50 mg/L of glycine, 20 mg/L of alanine, 50 mg/L of ascorbic acid, and 3 µg/L of CuSO₄ (all from Sigma, St. Louis, MO) at 37°C and 5% CO₂. Confluent cultures of HASMC between passage 4 and 8 were used in all studies.

### Endothelial cells

Primary human aortic endothelial cells (HAEC; GIBCO/Invitrogen Corp., Carlsbad, CA) were maintained and expanded in Medium 199 (Invitrogen Corp., Carlsbad, CA) supplemented with 10% FBS, 1 µg/mL of hydrocortisone, 10 ng/mL of human epidermal growth factor, 3 ng/mL of basic fibroblast growth factor, 10 µg/mL of heparin, 100 U/mL Penicillin, 0.1 mg/mL streptomycin, and 0.25 µg/mL of amphotericin B (all from Invitrogen Corp., Carlsbad, CA). The cells were grown on gelatin (from porcine serum; Sigma, St. Louis, MO) coated tissue culture treated flasks at 37°C and 5% CO₂. Confluent cultures of HAEC between passage 4 and 8 were used in all studies.

### Fibroblasts

Primary human dermal fibroblasts (HDF; GIBCO/Invitrogen Corp., Carlsbad, CA) were maintained and expanded in Medium 106 (Invitrogen Corp., Carlsbad, CA) supplemented with 2% FBS, 1 µg/mL of hydrocortisone, 10 ng/mL of human epidermal growth factor, 3 ng/mL of basic fibroblast growth factor, 10 µg/mL of heparin, 100 U/mL Penicillin, and 0.1 mg/mL streptomycin (all from Invitrogen Corp., Carlsbad, CA) at 37°C and 5% CO₂. Confluent cultures of HDF between passage 4 and 8 were used in all studies.

### SMC-like cells from the SVF of human lipoaspirate

SMC-like cells were generated from the adherent fraction of cells isolated after collagenase digestion of lipoaspirates. This digestion produces a population of cells known as the stromal vascular fraction (SVF). The cells of the SVF are optionally plated on standard tissue culture plastic and adherent cells are further selected via appropriate culture conditions. SMC-like cells from the SVF of adipose tissue lipoaspirates were maintained and expanded in high glucose dulbecco's modified eagle medium (DMEM; Invitrogen Corp., Carlsbad, CA) supplemented with 10% fetal bovine serum (FBS), 100 U/mL Penicillin, 0.1 mg/mL streptomycin, 0.25 µg/mL of amphotericin B, 0.01M of HEPES (all from Invitrogen Corp., Carlsbad, CA), 50 mg/L of proline, 50 mg/L of glycine, 20 mg/L of alanine, 50 mg/L of ascorbic acid, and 3 µg/L of CuSO₄ (all from Sigma, St. Louis, MO) at 37°C and 5% CO₂. Confluent subcultures of SVF-SMC between passage 3 and 8 were used in all studies.

### EC from the SVF of human lipoaspirate

Endothelial cells from the stromal vascular fraction (SVF) were maintained and expanded in growth media that is commonly used to grow primary isolates of bona fide endothelial cells (EC). Specifically, SVF-EC were maintained in M199 supplemented with 10% FBS, 1 µg/mL of hydrocortisone, 10 ng/mL of human epidermal growth factor, 3 ng/mL basic fibroblast growth factor, 10 µg/mL of heparin, 100 U/mL Penicillin, and 0.1 mg/mL streptomycin. The cells were grown on tissue culture-treated flasks at 37°C and 5% CO₂. Confluent cultures of SVF-EC between passage 3 and 8 were used in all studies.

### Example 2 - NovoGel™ solutions and mold

### Preparation of 2% and 4% (w/v) NovoGel™ solution

1 g or 2 g (for 2% or 4% respectively) of NovoGel™ (Organovo, San Diego, CA) was dissolved in 50 mL of Dulbecco's phosphate buffered saline (DPBS; Invitrogen Corp., Carlsbad, CA). Briefly, the DPBS and NovoGel™ are heated to 85°C on a hot plate with constant stirring until the NovoGel™ dissolves completely. NovoGel™ solution is sterilized by steam sterilization at 125°C for 25 minutes. The NovoGel™ solution remains in liquid phase as long as the temperature is maintained above 65.5°C. Below this temperature a phase transition occurs, the viscosity of the NovoGel™ solution increases and the NovoGel™ forms a solid gel.

### Preparation of NovoGel™ mold

An NovoGel™ mold was fabricated for the incubation of cylindrical bio-ink using a Teflon® mold that fit a 10 cm Petri dish. Briefly, the Teflon® mold was pre-sterilized using 70% ethanol solution and subjecting the mold to UV light for 45 minutes. The sterilized mold was placed on top of the 10 cm Petri dish (VWR International LLC, West Chester, PA) and securely attached. This assembly (Teflon® mold + Petri dish) was maintained vertically and 45 mL of pre-warmed, sterile 2% NovoGel™ solution was poured in the space between the Teflon® mold and the Petri dish. The assembly was then placed horizontally at room temperature for 1 hour to allow complete gelation of the NovoGel™. After gelation, the Teflon® print was removed and the NovoGel™ mold was washed twice using DPBS. Then 17.5 mL of HASMC culture medium was added to the NovoGel™ mold for incubating the polytypic cylindrical bio-ink.

### Example 3 - Fabrication of HASMC-HAEC polytypic cylindrical bio-ink

To prepare polytypic cylindrical bio-ink, HASMC and HAEC were individually collected and then mixed at pre-determined ratios. Briefly, the culture medium was removed from confluent culture flasks and the cells were washed with DPBS (1 ml/5 cm² of growth area). Cells were detached from the surface of the culture flasks by incubation in the presence of trypsin (1 ml/15 cm² of growth area; Invitrogen Corp., Carlsbad, CA) for 10 minutes. HASMC were detached using 0.15% trypsin while HAEC were detached using 0.1% trypsin. Following the incubation appropriate culture medium was added to the flasks (2X volume with respect to trypsin volume). The cell suspension was centrifuged at 200g for 6 minutes followed by complete removal of supernatant solution. Cell pellets were resuspended in respective culture medium and counted using a hemocytometer. Appropriate volumes of HASMC and HAEC were combined to yield a polytypic cell suspension containing 15% HAEC and remainder 85% HASMC (as a % of total cell population). The polytypic cell suspension was centrifuged at 200g for 5 minutes followed by complete removal of supernatant solution. Polytypic cell pellets were resuspended in 6 mL of HASMC culture medium and transferred to 20 mL glass vials (VWR International LLC, West Chester, PA), followed by incubation on a orbital shaker at 150 rpm for 60 minutes, and at 37°C and 5% CO₂. This allows the cells to aggregate with one another and initiate cell-cell adhesions. Post-incubation, the cell suspension was transferred to a 15 mL centrifuge tube and centrifuged at 200g for 5 minutes. After removal of the supernatant medium, the cell pellet was resuspended in 400 µl of HASMC culture medium and pipetted up and down several times to ensure all cell clusters were broken. The cell suspension was transferred into a 0.5 mL microfuge tube (VWR International LLC, West Chester, PA) placed inside a 15 mL centrifuge tube followed by centrifugation at 2000g for 4 minutes to form a highly dense and compact cell pellet. The supernatant medium was aspirated and the cells were transferred into capillary tubes (OD 1.5 mm, ID 0.5 mm, L 75 mm; Drummond Scientific Co., Broomall, PA) by aspiration so as to yield cylindrical bio-ink 50 mm in length. The cell paste inside the capillaries was incubated in HASMC medium for 20 minutes at 37°C and 5% CO₂. The cylindrical bio-ink was then extruded from the capillary tubes into the grooves of a NovoGel™ mold (*see,* e.g., **Example 2)** (covered with HASMC medium) using the plunger supplied with the capillaries. The cylindrical bio-ink was incubated for 24 hours at 37°C and 5% CO₂.

### Example 4 - Fabrication of HASMC-HDF-HAEC polytypic cylindrical bio-ink

To prepare polytypic cylindrical bio-ink, HASMC, HDF, and HAEC were individually collected and then mixed at pre-determined ratios (e.g., HASMC:HDF:HAEC ratios of 70:25:5). Briefly, the culture medium was removed from confluent culture flasks and the cells were washed with DPBS (1 ml/10 cm2 of growth area). Cells were detached from the surface of the culture flasks by incubation in the presence of trypsin (1 ml/15 cm2 of growth area; Invitrogen Corp., Carlsbad, CA) for 10 minutes. HASMC and HDF were detached using 0.15% trypsin while HAEC were detached using 0.1% trypsin. Following the incubation appropriate culture medium was added to the flasks (2X volume with respect to trypsin volume). The cell suspension was centrifuged at 200g for 6 minutes followed by complete removal of supernatant solution. Cell pellets were resuspended in respective culture medium and counted using a hemocytometer. Appropriate volumes of HASMC, HDF, and HAEC were combined to yield polytypic cell suspensions. The polytypic cell suspensions were centrifuged at 200g for 5 minutes followed by aspiration of the supernatant solution. Polytypic cell pellets were resuspended in 6 mL of HASMC culture medium and transferred to 20 mL glass vials (VWR International LLC, West Chester, PA), followed by incubation on a orbital shaker at 150 rpm for 60 minutes, and at 37°C and 5% CO₂. This allows the cells to aggregate with one another and initiate cell-cell adhesions. Post-incubation, the cell suspension was transferred to a 15 mL centrifuge tube and centrifuged at 200g for 5 minutes. After removal of the supernatant medium, the cell pellet was resuspended in 400 µL of HASMC culture medium and pipetted up and down several times to ensure all cell clusters were broken. The cell suspension was transferred into a 0.5 mL microfuge tube (VWR International LLC, West Chester, PA) placed inside a 15 mL centrifuge tube followed by centrifugation at 2000g for 4 minutes to form a highly dense and compact cell pellet. The supernatant medium was aspirated and the cells were transferred into capillary tubes (OD 1.25 mm, ID 0.266 mm, L 75 mm; Drummond Scientific Co., Broomall, PA) by aspiration so as to yield cylindrical bio-ink 50 mm in length. The cell paste inside the capillaries was incubated in HASMC medium for 20 minutes at 37°C and 5% CO₂. The cylindrical bio-ink was then extruded from the capillary tubes into the grooves of a NovoGel™ mold (covered with HASMC medium) using the plunger supplied with the capillaries. The cylindrical bio-ink was incubated for 6 to 24 hours at 37°C and 5% CO₂.

### Example 5 - Fabrication of SVF-SMC-SVF-EC polytypic cylindrical bio-ink

To prepare polytypic cylindrical bio-ink, SVF-SMC and SVF-EC were individually collected and then mixed at pre-determined ratios. Briefly, the culture medium was removed from confluent culture flasks and the cells were washed with DPBS (1 ml/5 cm² of growth area). Cells were detached from the surface of the culture flasks by incubation in the presence of TrypLE (Invitrogen Corp., Carlsbad, CA) for 5 to 10 minutes. Following the incubation appropriate culture medium was added to the flasks to quench enzyme activity. The cell suspension was centrifuged at 200g for 6 minutes followed by complete removal of supernatant solution. Cell pellets were resuspended in respective culture medium and counted using a hemocytometer. Appropriate volumes of SVF-SMC and SVF-EC were combined to yield a polytypic cell suspension containing 15% SVF-EC and remainder 85% SVF-SMC (as a % of total cell population). The polytypic cell suspension was centrifuged at 200g for 5 minutes followed by complete removal of supernatant solution. Polytypic cell pellets were resuspended in 6 mL of SVF-SMC culture medium and transferred to 20 mL glass vials (VWR International LLC, West Chester, PA), followed by incubation on a orbital shaker at 150 rpm for 60 minutes, and at 37°C and 5% CO₂. This allows the cells to aggregate with one another and initiate cell-cell adhesions. Post-incubation, the cell suspension was transferred to a 15 mL centrifuge tube and centrifuged at 200g for 5 minutes. After removal of the supernatant medium, the cell pellet was resuspended in 400 µl of SVF-SMC culture medium and pipetted up and down several times to ensure all cell clusters were broken. The cell suspension was transferred into a 0.5 mL microfuge tube (VWR International LLC, West Chester, PA) placed inside a 15 mL centrifuge tube followed by centrifugation at 2000g for 4 minutes to form a highly dense and compact cell pellet. The supernatant medium was aspirated and the cells were transferred into capillary tubes (OD 1.25 mm, ID 0.266 mm, L 75 mm; Drummond Scientific Co., Broomall, PA) by aspiration so as to yield cylindrical bio-ink 50 mm in length. The cell paste inside the capillaries was incubated in SVF-SMC medium for 20 minutes at 37°C and 5% CO₂. The cylindrical bio-ink was then extruded from the capillary tubes into the grooves of a NovoGel™ mold (covered with SVF-SMC medium) using the plunger supplied with the capillaries. The cylindrical bio-ink was incubated for 6 to 12 hours at 37°C and 5% CO₂.

### Example 6 - Bioprinting blood vessel wall segments comprising a mixture of vascular SMC and EC

Blood vessel wall constructs were bioprinted utilizing a NovoGen MMX Bioprinter™ (Organovo, Inc., San Diego, CA) into the wells of 6-well culture plates that had been previously covered with 1.5 mL of 2% (w/v) NovoGel™. Cellular bio-ink cylinders were prepared with a mixture of human vascular smooth muscle cells (SMC) and human endothelial cells (EC) at an SMC:EC ratio of 85:15 or 70:30. Bio-ink cylinders were generated by aspiration of a cell pellet (SMC:EC) into a glass microcapillary tube with either a 500 µm or 266 µm inner diameter (ID). The bio-ink cylinders were then extruded into a NovoGel™ mold covered with appropriate culture medium. Prior to bioprinting, the cylindrical bio-ink was held for 6 to 18 hours. Cylinders containing a mixture of SMC and EC were used. In these experiments the EC within the cylinders sorted to the periphery of the cylinders resulting in a construct that is covered with EC and contains a SMC-rich construct wall. This process resulted in the development of a smooth muscle construct that contains a wall comprised of SMC and a covering of EC. The constructs were bioprinted in the center of the culture well using bioprinting protocols and the culture well was filled with appropriate culture media and the constructs returned to the incubator for maturation and evaluation. Following bioprinting, the construct was covered with an appropriate amount of culture media (e.g., 4 mL for 1 well of a 6-well plate). In summary, this example describes the use of vascular SMC and EC for bioprinting a small-scale smooth muscle construct within a standard size multi-well tissue culture plate. The resulting smooth muscle construct is characterized by an external layer or layers of EC and internal wall comprised largely or solely of SMC.

### Example 7 - Bioprinting blood vessel wall segments comprising human vascular SMC with a covering of EC

Blood vessel wall constructs were bioprinted utilizing a NovoGen MMX Bioprinter™ (Organovo, Inc., San Diego, CA) into the wells of 6-well culture plates that had been previously covered with 1.5 mL of 2% (w/v) NovoGel™. Cellular bio-ink cylinders were prepared with human vascular smooth muscle cells (SMC). Bio-ink cylinders were generated by aspiration of a cell pellet (SMC) into a glass microcapillary tube with either a 500 µm or 266 µm inner diameter (ID). The bio-ink cylinders were then extruded into a NovoGel™ mold covered with appropriate culture medium. Prior to bioprinting, the cylindrical bio-ink was held for 6 to 18 hours. An EC-concentrate (1-1.5x10⁵ cells/µl) was bioprinted directly on top of the previously bioprinted SMC structure. This process resulted in the development of a smooth muscle construct that contains a wall comprised of SMC and a covering of EC. The constructs were bioprinted in the center of the culture well using bioprinting protocols. Following bioprinting, the construct was covered with an appropriate amount of culture media (e.g., 4 mL for 1 well of a 6-well plate) and returned to the incubator for maturation and evaluation. In summary, this example describes the use of vascular SMC and EC for bioprinting a smooth muscle construct within a standard size multi-well tissue culture plate. The resulting smooth muscle construct is characterized by an external layer of EC and internal wall comprised largely or solely of SMC.

### Example 8 - Bioprinting blood vessel wall segments comprising HASMC layered with HAEC utilizing NovoGel™ containment

Blood vessel wall-mimicking segments were bioprinted utilizing a NovoGen MMX Bioprinter™ (Organovo, Inc., San Diego, CA) either inside NovoGel™ coated wells or directly onto Corning® Transwell® inserts in a multi-well plate (e.g., 6-well plates). This process involved the following three phases:

### Preparation of HASMC cylinders

Cultures of human aortic smooth muscle cells (HASMC) were trypsinized, and then shaken for 60 minutes on a rotary shaker. Post-shaking, cells were collected, centrifuged, and aspirated into 266 or 500 µm (ID) glass microcapillaries. Finally, the cells were extruded into media covered NovoGel™ plates and incubated for a minimum of 6 hours.

### Bioprinting of HASMC patches layered with HAEC

Just prior to bioprinting of patches (e.g., segments), human aortic endothelial cell (HAEC) cultures were trypsinized, counted, and then resuspended in HAEC medium at a working concentration of 1x10⁶ cells/10 µL of medium. The HAEC suspension was placed in the bioprinter to be utilized for layering bioprinted patches. In the case of printing onto NovoGel™ beds inside the wells of a multi-well plate, a first layer of NovoGel™ cylinders was bioprinted. Then, on top of it a box was bioprinted using NovoGel™ rods such that the space inside was 8 mm long x 1.25 mm wide. Matured HASMC cylinders at the end of the incubation period from above were re-aspirated into the microcapillaries and loaded onto the bioprinter for printing inside the box. HAEC in suspension were then drawn into a clean microcapillary by the bioprinter and dispensed on top of the printed HASMC cylinders 4 times near the 4 corners of the printed patch. Each drop was 2.5 µL in volume. The construct was incubated for a period of 15-30 minutes before proceeding to print the third layer. Finally, a third layer of NovoGel™ cylinders was printed on top of the second to create a lattice/mesh type structure on top. In the case of printing onto Transwell® inserts inside the wells of the plate, the first layer of NovoGel™ rods described earlier was eliminated. The bioprinted constructs were then covered with appropriate cell culture medium and incubated.

### Maturation of bioprinted constructs

The bioprinted constructs were incubated for a period of 1-7 days to allow the construct to mature and provide the HAEC sufficient time to form a uniformly thin monolayer on top of the HASMC patch. In some experiments, the three-dimensional smooth muscle patch was subjected to shear forces (i.e., pulsatile flow).

### Example 9 - Bioprinting blood vessel wall segments comprising HASMC layered with HAEC onto a HDFa monolayer utilizing NovoGel™ containment

Smooth muscle constructs were bioprinted utilizing a NovoGen MMX Bioprinter™ (Organovo, Inc., San Diego, CA) directly onto Corning® Transwell® inserts in a multi-well plate (e.g., 6-well plates). This process involved the following four phases:

### Culture of HDFa's onto Transwell® membranes

Human adult dermal fibroblasts (HDFa) were seeded onto Transwell® membranes at a density of 20,000 cells/cm² and cultured for a minimum of 6 days. This allowed the cells to adhere, grow and become a confluent layer on the Transwell® membrane.

### Preparation of HASMC cylinders

Cultures of human aortic smooth muscle cells (HASMC) were trypsinized, and shaken for 60 minutes on a rotary shaker. Post-shaking, cells were collected, centrifuged, and aspirated into 266 or 500 µm (ID) glass microcapillaries. The cells were then extruded into media covered NovoGel™ plates and incubated for a minimum of 6 hours.

### Bioprinting of HASMC patches layered with HAEC

Just prior to bioprinting of patches (e.g., segments), human aortic endothelial cell (HAEC) cultures were trypsinized, counted, and then resuspended in HAEC medium at a working concentration of 1x10⁶ cells/10 µL of medium. The HAEC suspension was placed in the bioprinter to be utilized for layering bioprinted patches. The culture media in the multi-well plates having the HDFa's grown on Transwell® membranes was completely aspirated and the plate transferred to the bioprinter. A box was bioprinted using NovoGel™ rods such that the space defined was 8 mm long x 1.25 mm wide directly on top of the HDFa's on the membrane. Matured HASMC cylinders at the end of the incubation period from above were re-aspirated into the microcapillaries and loaded onto the bioprinter for printing inside the box. HAEC in suspension were then drawn into a clean microcapillary tube by the bioprinter and dispensed on top of the printed HASMC cylinder 4 times near the 4 corners of the printed patch. Each drop was 2.5 µL in volume. The construct was incubated for a period of 15-30 minutes before proceeding to print the top NovoGel™ rod layer. Finally, a top layer of NovoGel™ cylinders was printed to create a lattice/mesh type structure. The bioprinted constructs were then covered with appropriate cell culture medium and incubated.

### Maturation of bioprinted constructs

The bioprinted constructs were incubated for a period of 1-7 days to allow the construct to mature and provide the HAEC sufficient time to form a uniformly thin monolayer on top of the HASMC patch.

### Example 10 - Bioprinting smooth muscle constructs comprising HASMC and HAEC polytypic bio-ink

Smooth muscle constructs were bioprinted utilizing a NovoGen MMX Bioprinter™ (Organovo, Inc., San Diego, CA) either on NovoGel™ base plates (100 mm Petri dish size), inside NovoGel™ coated wells, or directly onto Corning® Transwell® inserts in a multi-well plate (e.g., 6-well plates). This process involves the following three phases:

### Preparation of HASMC-HAEC polytypic bio-ink

Cultures of human aortic smooth muscle cells (HASMC) and human aortic endothelial cells (HAEC) were trypsinized, counted, and mixed in appropriate quantities to yield cylinders that comprised HASMC:HAEC at either a 85:15 or 70:30 ratio. The polytypic cell suspension was shaken for 60 minutes on a rotary shaker, collected, and centrifuged. Cells were drawn into 266 or 500 µm (ID) glass microcapillaries, then extruded into media covered NovoGel™ plates and incubated for a minimum of 6 hours.

### Bioprinting of patches / three-dimensional smooth muscle sheets

In the case of printing onto NovoGel™ beds inside the wells of a multi-well plate or on NovoGel™ base plates (100 mm Petri dish size), a first layer of NovoGel™ cylinders was bioprinted. Then, on top of it a box was bioprinted using NovoGel™ rods such that the space inside was 8 mm long x 1.25 mm wide. Matured polytypic cylindrical bio-ink at the end of the incubation period from above was re-aspirated into the microcapillaries and loaded onto the bioprinter for printing inside the box. Finally, a third layer of NovoGel™ cylinders was printed on top of the second that either covers the entire length of cells or creates a lattice/mesh type structure on top. In the case of printing onto Transwell® inserts inside the wells of the plate, the first layer of NovoGel™ rods described earlier was eliminated. The bioprinted constructs were then covered with appropriate cell culture medium and incubated during which the adjoining segments of the extruded bio-ink fused to form a three-dimensional patch of cells.

### Maturation of bioprinted constructs

The bioprinted constructs comprising the HASMC-HAEC polytypic bio-ink were incubated for a period of 1-7 days to allow the construct to mature and provide the HAEC sufficient time to sort to the periphery of the construct thereby yielding a smooth muscle construct with a layer comprising a second cell type (endothelial cells, in this example). In some experiments, the three-dimensional smooth muscle patch was subjected to shear forces (i.e., pulsatile flow) to aid the process of HAEC sorting.

### Example 11 - Bioprinting blood vessel wall segments comprising HASMC, HAEC, and HDFa polytypic cylindrical bio-ink

Smooth muscle constructs were bioprinted utilizing a NovoGen MMX Bioprinter™ (Organovo, Inc., San Diego, CA) either on NovoGel™ base plates (100 mm Petri dish size), inside NovoGel™ coated wells, or directly onto Corning® Transwell® inserts in a multi-well plate (e.g., 6-well plates). This process involves the following three phases:

### Preparation of HASMC-HDFa-HAEC polytypic bio-ink

Cultures of HASMC, HAEC, and HDFa were trypsinized, counted, and mixed in appropriate quantities to yield cylindrical bio-ink that comprised HASMC:HDFa:HAEC at a 70:15:15 ratio. The polytypic cell suspension was shaken for 60 minutes on a rotary shaker, collected, and centrifuged. Cells were drawn into 266 or 500 µm (ID) glass microcapillaries, then extruded into media covered NovoGel™ plates and incubated for a minimum of 6 hours.

### Bioprinting of patches / three-dimensional cell sheets

In the case of printing onto NovoGel™ beds inside the wells of a multi-well plate or on NovoGel™ base plates (100 mm Petri dish size), a first layer of NovoGel™ cylinders was bioprinted. Then, on top of it a box was bioprinted using NovoGel™ rods such that the space inside was 8 mm long x 1.25 mm wide. Matured polytypic cylindrical bio-ink at the end of the incubation period from above was re-aspirated into the microcapillaries and loaded onto the bioprinter for printing inside the box. Finally, a third layer of NovoGel™ cylinders was printed on top of the second that either covers the entire length of cells or creates a lattice/mesh type structure on top. In the case of printing onto Transwell® inserts inside the wells of the plate, the first layer of NovoGel™ rods described earlier was eliminated. The bioprinted constructs were then covered with appropriate cell culture medium and incubated during which the adjoining segments of the cell cylinder fused to form a three-dimensional patch of cells.

### Maturation of bioprinted constructs

The bioprinted constructs comprising the HASMC-HDFa-HAEC polytypic bio-ink were incubated for a period of 1-7 days to allow the constructs to mature and provide the HAEC sufficient time to sort to the periphery of the construct thereby yielding a smooth muscle construct with layer(s) representing other cell types (endothelial cells and fibroblasts, in this example). In some experiments, the three-dimensional smooth muscle construct was subjected to shear forces (i.e., pulsatile flow) to aid the process of HAEC sorting.

### Example 12 - Hydrogel lattice used to spatially confine a construct while allowing for direct contact with media

Cylindrical hydrogel elements were dispensed utilizing a NovoGen MMX Bioprinter™ (Organovo, Inc., San Diego, CA) across a portion of the top surface of a three-dimensional smooth muscle construct. The lattice provided spatial confinement to the bioprinted tissue and allowed for direct contact between the construct and the surrounding media. First, a hydrogel base layer was dispensed. Second, a hydrogel window was dispensed defining a space 8 mm long x 1.25 mm wide. Third, smooth muscle bio-ink was bioprinted inside the hydrogel window to form the three-dimensional cell sheet. And, fourth, the hydrogel lattice structure was dispensed. In various experiments, the size of the hydrogel elements was approximately 100 µm to 1 mm in diameter, and the spacing between the elements was approximately 100 µm to 10 mm.

In some experiments, the hydrogel elements were dispensed along one direction to create long open channels on top of the smooth muscle sheet. In other experiments, the hydrogel elements were dispensed in multiple directions to create a grid-like pattern of open areas on top of the sheet. The hydrogel was comprised of NovoGel™. The lattice structure was optionally extended past the structure and onto the support surface to allow for the application of additional material to affix the structure to the print surface. The resulting lattice was used to spatially confine the construct, but allow for some of the cellular construct to have direct contact with the surrounding nutritive media.

### Example 13 - Bioprinting implantable tubes (not forming part of the invention), sheets, and sacs (not forming part of the invention) without use of synthetic polymer or exogenous extracellular matrix

Human smooth muscle cells (SMC) were cultured from native SMC tissue sources or generated from the stromal vascular fraction (SVF) of adipose tissue and utilized to generate bio-ink. The bio-ink comprised self-assembled aggregates of cells, 180-500 µm in diameter, in either spherical or cylindrical form. The bio-ink was loaded onto a NovoGen MMX Bioprinter™ (Organovo, Inc., San Diego, CA) and used to build three-dimensional structures layer by layer. Within 24-72 hours, the bioprinted structures fused to generate stable tubes or thick sheets comprised of SMCs. In some cases, fibroblasts, endothelial cells, or epithelial cells were incorporated in admixture with the SMC, or as specific layers or components of the bioprinted construct. In some experiments, additional cell layers of endothelial cells or tissue-specific epithelial cells were applied post-printing. In some cases, the bioprinted construct was subjected to specific biomechanical or biochemical conditioning to facilitate specification of the construct toward a targeted application. The resulting constructs recapitulated human tissue architecture and generated sufficient extracellular matrix *in situ* that they could be handled and manipulated as solid tissues.

### Example 14 - Liver tissue bioprinted using continuous deposition and multi-layered, tessellated geometry

Engineered liver tissue was bioprinted utilizing a NovoGen MMX Bioprinter™ (Organovo, Inc., San Diego, CA) using a continuous deposition mechanism. The three-dimensional structure of the liver tissue was based on a functional unit repeating in a planar geometry, in this case, a hexagon. The bio-ink was composed of hepatic stellate cells and endothelial cells encapsulated in an extrusion compound (surfactant polyol - PF-127).

### Preparation of 30% PF-127

A 30% PF-127 solution (w/w) was made using PBS. PF-127 powder was mixed with chilled PBS using a magnetic stir plate maintained at 4°C. Complete dissolution occurred in approximately 48 hours.

### Cell Preparation and bioprinting

A cell suspension comprised of 82% stellate cells (SC) and 18% human aortic endothelial cells (HAEC) and human adult dermal fibroblasts (HDFa) was separated into 15 mL tubes in order to achieve three cell concentrations: 50 x 10⁶ cells/mL, 100 x 10⁶ cells/mL, and 200 x 10⁶ cells/mL following centrifugation. Each cell pellet was resuspended in 30% PF-127 and aspirated into a 3 cc reservoir using the bioprinter. With a 510 µm dispense tip, the encapsulated cells were bioprinted onto a PDMS base plate into a single hexagon (*see* **Fig. 7A****)** or hexagon tessellation configuration (*see* **Fig. 7B****).** Each construct received approximately 200 µL of media and was incubated for 20 minutes at room temperature to evaluate construct integrity.

### Multi-layer bioprinting

For hexagon tessellation experiments, up to (4) sequential layers were bioprinted resulting in a taller structure with more cellular material present. Following fabrication, each construct initially received approximately 200 µL of complete media to assess construct integrity. Constructs were incubated for 20 minutes at room temperature and were then submerged in 20 mLs of complete media.

### Results

Following 18 hours of culture in growth media containing 10% fetal bovine serum (which dissolves PF127), cells contained within the bioprinted geometry were cohered to each other sufficiently to generate an intact, contiguous sheet of tissue that retained the geometrical patterning of the original design *(see* **Fig. 7D****).** Shown in **Fig. 8** is H&E staining of a single segment of the tessellated construct, after fixation in 10% neutral buffered formalin. Cells were found to be viable, intact, and confined to their original printed geometry.

### Example 15 - Planar geometry in a multi-layered bioprinted tissue patch

Bio-ink was formed as previously described into cylindrical, stable cellular aggregates, typically 250 µm or 500 µm in diameter. Briefly, cells were propagated under typical laboratory conditions and when cells achieved 70%-80% confluence they were detached from the cell culture surface through the application of 0.1% Trypsin without EDTA. Following trypsinization, cells were washed once in serum-containing media, collected, counted and centrifuged to form a large cell pellet. Cell pellets were either aspirated into capillaries for generating homogeneous (i.e., monotypic) bio-ink, or resuspended in order to create user-defined cell mixtures (*see* **Table 1,** below) yielding heterogeneous (i.e., polytypic) complex bio-ink admixtures. *See,* e.g., **Fig. 9****.** Bio-ink cylinders created in this fashion are optionally utilized directly for bioprinting tubular constructs.

**Table 1**

| **Putative Bio-ink Compositions (%)** | **Potential cell types** | **Working prototypes (%)** |
|---|---|---|
| 100 (monotypic, n=1) | Smooth muscle cells (SMCs) | 100, SMCs |
| 30:70 (polytypic, n=2) | Epithelial cells (Epi) | 30:70, SMC:Fib |
| 50:50 (polytypic, n=2) | Fibroblasts (Fib) | 70:30, SMC:Fib |
| 5:25:70 (polytypic, n=3) | Endothelial cells (ECs) | 5:25:70, EC:SMC:Fib |
| 5:20:75 (polytypic, n=3) | Monocytes/Macrophages | 5:25:70, EC:Fib:SMC |
| 10:30:60 (polytypic, n=3) | Stellate cells | 5:25:70, Epi:Fib:SMC |
| 10:10:10:70 (polytypic, n=4) | Hepatocytes | 5:25:70, Epi:SMC:Fib |
| 25:25:25:25 (polytypic, n=4) | Osteocytes | 50:50 SMC:Fib |

**Table 1** is an incomplete list of bio-ink formulations based on cellular composition is presented. Formulations optionally consist of either single cell types or admixtures of different cell types at a variety of proportions in order to address native tissue architecture and/or cellular reorganization in bioprinted neo-tissues. Putative bio-ink compositions are expressed as percent composition with a listing of cell types that have been examined and numerous prototypes that have been created.

The working prototypes enumerated in **Table 1** are optionally generated in a variety of different sizes based on the intended targeted application of the tubular construct (not forming part of the invention). For example, several commonly-utilized schemas for tubular structures are presented in cross-section in **Fig.** 10 (not forming part of the invention).

**Fig. 11** demonstrates a 6/1 working prototype tubular construct bioprinted with bio-ink consisting of 70:30 SMC Fib (not forming part of the invention).

Implantable tubular tissues (not forming part of the invention) of a variety of cell mixtures, but in particular, smooth muscle cell (SMCs) components provide suitable composition and functional characteristics for application in numerous target locations within the body. Some exemplary applications include respiratory grafts, gastrointestinal grafts, and urological grafts.

In some embodiments, implantable bioprinted sheets are surgically attached by either a continuous running suture or multiple interrupted sutures. *See* **Fig. 12****.**

### Example 16 - Bioprinted Skeletal Muscle Patches

Cellular bio-ink cylinders were prepared with a myoblast cell line (C2C12), human aortic endothelial cells (HAEC), and/or human dermal fibroblasts (HDFa). Cells were propagated under standard laboratory conditions with media comprised of components typically found in the primary literature to be conducive to standard cell culture practices for those particular cell types. Once the desired confluence was reached (typically 60-100%), cells were liberated from the standard tissue culture plastic by washing with cation-free phosphate buffered saline (PBS) and then exposed to 0.05% - 0.1% trypsin (Invitrogen). Liberated cells were washed in serum-containing media, collected, counted, combined in an appropriate ratio, and pelleted by centrifugation. Typically, C2C12 were mixed in the following ratios: 100% C2C12, 90:10 (C2C12:HAEC), 90:10 (C2C12:HDFa), or 80:10:10 (C2C12:HAEC:HDFa). The supernatant was removed and cells were resuspended in fibrinogen (2 mg/mL). The cell mixture was pelleted by centrifugation, supernatant was removed from the cell pellet, and the cell mixture was aspirated into a glass capillary of a desired diameter, typically 250 or 500 µm. Following a 15-20 minute submersion in media, the contents of each capillary were extruded into a non-adherent hydrogel mold containing linear channels and incubated in media for 4 to 24 hours.

Skeletal muscle constructs were then bioprinted onto the membrane of a cell culture well insert (Transwell®, BD) using the cellular bio-ink cylinders containing C2C12, HAEC, and/or HDFa. Skeletal muscle tissue segments were fabricated with initial dimensions of 1.25 mm x 8.00 mm x 0.25 mm (W x L x H). Following fabrication, the skeletal muscle patches were submerged in complete serum-containing cell culture media and placed in a standard humidified chamber, supplemented with 5% CO₂ for maturation. The bioprinted skeletal muscle segments were then cultured in static conditions or stimulated through the addition of cytokine(s) or biomechanical signals. Bioprinted skeletal muscle constructs were cultured for up to nine days and evaluated for cell organization, extracellular matrix production, cell viability, and construct integrity. *See,* e.g., **Figs. 13A****, B,** and **C.**

### Results

Bioprinted skeletal muscle tissue constructs comprising of C2C12, HAEC, and/or HDFa were successfully fabricated and maintained in culture.

## Claims

1. A living, three-dimensional engineered tissue or organ comprising a plurality of layers, the plurality of layers **characterized by** being:
a) substantially free of pre-formed scaffold at the time of bioprinting and at the time of use; and
b) having at least one component bioprinted by extrusion of a bio-ink comprising a plurality of cells, the bio-ink being a solid or semi-solid composition, onto a biocompatible surface; the plurality of layers suitable for implantation in a vertebrate subject upon sufficient maturation;
provided that at least one layer of the engineered tissue or organ comprises muscle cells and that the engineered tissue or organ is a sheet and is not a vascular tube, and the plurality of layers are cohered to one another.

2. A tissue or organ as claimed in claim 1, wherein at least one layer comprises a plurality of cell types, the cell types spatially arranged relative to each other to create a planar geometry.

3. A tissue or organ as claimed in claim 1, wherein at least one layer compositionally or architecturally distinct from at least one other layer to create a laminar geometry.

4. A tissue or organ as claimed in any preceding claim, wherein the plurality of layers generates an extracellular matrix.

5. A tissue or organ as claimed in any preceding claim, wherein the muscle cells are smooth muscle cells.

6. A tissue or organ as claimed in any preceding claim, wherein the muscle cells were derived from stem cells or progenitor cells capable of differentiating into the muscle cells.

7. A tissue or organ as claimed in any preceding claim, further comprising cells selected from: endothelial cells, nerve cells, pericytes, fibroblasts, tissue-specific epithelial cells, chondrocytes, skeletal muscle cells, cardiomyocytes, bone-derived cells, soft tissue-derived cells, mesothelial cells, tissue-specific stromal cells, stem cells, progenitor cells, endoderm-derived cells, ectoderm-derived cells, mesoderm-derived cells, and combinations thereof.

8. A tissue or organ as claimed in any preceding claim, wherein cells other than muscle cells were dispensed on at least one surface of the plurality of layers.

9. A tissue or organ as claimed in claim 8, wherein cells other than muscle cells were bioprinted on at least one surface of the plurality of layers.

10. A tissue or organ as claimed in claim 9, wherein the cells other than muscle cells were dispensed on the plurality of layers at substantially the same time the plurality of layers was fabricated, following fabrication of the plurality of layers, during maturation of the plurality of layers, or following maturation of the plurality of layers.

11. A tissue or organ as claimed in claim 1, wherein the tissue or organ is a muscle cell-comprising sheet or patch suitable for wound repair, tissue replacement, or tissue augmentation.

12. A method for making a three-dimensional, engineered, implantable tissue or organ comprising a plurality of layers, the method comprising:
a. bioprinting at least one component by extruding bio-ink which is a solid or semi-solid composition comprising muscle cells into a form onto a biocompatible surface; and
b. fusing the bio-ink into a cohesive cellular structure;
wherein the implantable tissue or organ is substantially free of any pre-formed scaffold at the time of bioprinting and at the time of use, and provided that the tissue or organ is implantable in a vertebrate subject, is a sheet and is not a vascular tube, and the plurality of layers are cohered to one another.

13. A method as claimed in claim 12, wherein the bio-ink further comprises cells selected from: endothelial cells, nerve cells, pericytes, fibroblasts, tissue-specific epithelial cells, chondrocytes, skeletal muscle cells, cardiomyocytes, bone-derived cells, soft tissue- derived cells, mesothelial cells, tissue-specific stromal cells, stem cells, progenitor cells, endoderm-derived cells, ectoderm-derived cells, mesoderm-derived cells, and combinations thereof.

14. A method as claimed in claim 12, further comprising the step of bioprinting, spraying, painting, applying, dip coating, grafting, seeding, injecting, or layering cells of claim 13 into or onto the bioprinted form of claim 12.

## Patentansprüche

1. Lebendes, dreidimensional konstruiertes Gewebe oder ein Organ, das eine Mehrzahl von Schichten umfasst, wobei die Mehrzahl von Schichten **dadurch gekennzeichnet ist, dass** für sie folgendes gilt:
a) sie sind im Wesentlichen frei von einem vorgeformten Gerüst zum Zeitpunkt des Biodrucks und zum Zeitpunkt der Verwendung; und
b) sie weisen mindestens eine durch Extrusion einer Biotinte auf eine biokompatible Oberfläche biogedruckte Komponente auf, die eine Mehrzahl von Zellen umfasst, wobei die Biotinte eine feste oder halbfeste Zusammensetzung ist; wobei die Mehrzahl von Schichten nach ausreichender Maturation geeignet ist für eine Implantation in ein Wirbeltiersubjekt;
vorausgesetzt, dass mindestens eine Schicht des konstruierten Gewebes oder Organs Muskelzellen umfasst, und dass das konstruierte Gewebe oder Organ eine Lage ist und keine Gefäßröhre ist und dass die Mehrzahl von Schichten zusammenhängen.

2. Gewebe oder Organ nach Anspruch 1, wobei mindestens eine Schicht eine Mehrzahl von Zellarten umfasst, wobei die Zellarten räumlich im Verhältnis zueinander angeordnet sind, so dass eine planare Geometrie erzeugt wird.

3. Gewebe oder Organ nach Anspruch 1, wobei sich mindestens eine Schicht von der Zusammensetzung oder vom Aufbau her von mindestens einer anderen Schicht unterscheidet, so dass eine laminare Geometrie erzeugt wird.

4. Gewebe oder Organ nach einem der vorstehenden Ansprüche, wobei die Mehrzahl von Schichten eine extrazelluläre Matrix erzeugt.

5. Gewebe oder Organ nach einem der vorstehenden Ansprüche, wobei die Muskelzellen glatte Muskelzellen sind.

6. Gewebe oder Organ nach einem der vorstehenden Ansprüche, wobei die Muskelzellen aus Stammzellen oder Vorläuferzellen stammen, die in die Muskelzellen differenziert werden können.

7. Gewebe oder Organ nach einem der vorstehenden Ansprüche, ferner Zellen umfassend, die ausgewählt sind aus: Endothelzellen, Nervenzellen, Perizyten, Fibroblasten, gewebespezifischen Endothelzellen, Chondrozyten, Skelettmuskelzellen, Cardiomyozyten, aus Knochen stammenden Zellen, aus Weichgewebe stammenden Zellen, Mesothelzellen, gewebespezifischen Stromalzellen, Stammzellen, Vorläuferzellen, Endodermzellen, Ektodermzellen, Mesodermzellen und Kombinationen dieser.

8. Gewebe oder Organ nach einem der vorstehenden Ansprüche, wobei andere Zellen als Muskelzellen auf mindestens einer Oberfläche der Mehrzahl von Schichten verteilt worden sind.

9. Gewebe oder Organ nach Anspruch 8, wobei andere Zellen als Muskelzellen auf mindestens einer Oberfläche der Mehrzahl von Schichten biogedruckt worden sind.

10. Gewebe oder Organ nach Anspruch 9, wobei die anderen Zellen als Muskelzellen auf der Mehrzahl von Schichten im Wesentlichen gleichzeitig verteilt worden sind wie die Mehrzahl von Schichten hergestellt worden sind, nach der Herstellung der Mehrzahl von Schichten, während der Maturation der Mehrzahl von Schichten oder nach der Maturation der Mehrzahl von Schichten.

11. Gewebe oder Organ nach Anspruch 1, wobei das Gewebe oder Organ eine Lage oder ein Patch ist, das Muskelzellen umfasst, geeignet für die Wundheilung, einen Gewebeersatz oder eine Gewebeverstärkung.

12. Verfahren zur Herstellung eines lebenden, dreidimensional konstruierten Gewebes oder eines Organs, das eine Mehrzahl von Schichten umfasst, wobei das Verfahren folgendes umfasst:
a) Biodrucken mindestens einer Komponente durch Extrudieren von Biotinte, die eine feste oder halbfeste Zusammensetzung ist, die Muskelzellen umfasst, in eine Form auf einer biokompatiblen Oberfläche; und
b) Verschmelzen der Biotinte in eine zusammenhängende Zellstruktur;
wobei das implantierbare Gewebe oder Organ im Wesentlichen frei ist von einem vorgeformten Gerüst zum Zeitpunkt des Biodrucks und zum Zeitpunkt der Verwendung, und vorausgesetzt, dass das in ein Wirbeltiersubjekt implantierbare Gewebe oder Organ eine Lage ist und keine Gefäßröhre ist und dass die Mehrzahl von Schichten zusammenhängen.

13. Verfahren nach Anspruch 12, wobei die Biotinte ferner Zellen umfasst, die ausgewählt sind aus: Endothelzellen, Nervenzellen, Perizyten, Fibroblasten, gewebespezifischen Endothelzellen, Chondrozyten, Skelettmuskelzellen, Cardiomyozyten, aus Knochen stammenden Zellen, aus Weichgewebe stammenden Zellen, Mesothelzellen, gewebespezifischen Stromalzellen, Stammzellen, Vorläuferzellen, Endodermzellen, Ektodermzellen, Mesodermzellen und Kombinationen dieser.

14. Verfahren nach Anspruch 12, wobei dieses ferner den Schritt des Biodruckens, Sprühens, Streichens, Auftragens, Tauchüberziehens, Pfropfens, Impfens, Injizierens oder Schichtens von Zellen nach Anspruch 13 in oder auf die biogedruckte Form nach Anspruch 12 umfasst.

## Revendications

1. Tissu ou organe artificiel tridimensionnel vivant comprenant une pluralité de couches, la pluralité de couches étant **caractérisée en ce que** :
a) elle est sensiblement exempte d'échafaudage préformé au moment de la bio-impression et au moment de l'utilisation ; et
b) elle a au moins un composant bio-imprimé par extrusion d'une bio-encre comprenant une pluralité de cellules, la bio-encre étant une composition solide ou semi-solide, sur une surface biocompatible ; la pluralité de couches appropriées pour l'implantation chez un sujet vertébré après maturation suffisante ; pourvu qu'au moins une couche du tissu ou de l'organe modifié comprenne des cellules musculaires et que le tissu ou l'organe modifié soit une feuille et non un tube vasculaire et la pluralité des couches soit cohérente entre elles.

2. Tissu ou organe selon la revendication 1, au moins une couche comprenant une pluralité de types de cellules, les types de cellules étant disposés spatialement les uns par rapport aux autres pour créer une géométrie plane.

3. Tissu ou organe selon la revendication 1, au moins une couche étant distincte sur le plan de la composition ou de l'architecture d'au moins une autre couche pour créer une géométrie laminaire.

4. Tissu ou organe selon l'une quelconque des revendications précédentes, la pluralité de couches générant une matrice extracellulaire.

5. Tissu ou organe selon l'une quelconque des revendications précédentes, les cellules musculaires étant des cellules musculaires lisses.

6. Tissu ou organe selon l'une quelconque des revendications précédentes, les cellules musculaires étant dérivées de cellules-souches ou de cellules progénitrices pouvant se différencier en cellules musculaires.

7. Tissu ou organe selon l'une quelconque des revendications précédentes, comprenant en outre des cellules sélectionnées parmi : des cellules endothéliales, des cellules nerveuses, des péricytes, des fibroblastes, des cellules épithéliales spécifiques de tissu, des chondrocytes, des cellules musculaires squelettiques, des cardiomyocytes, des cellules dérivées d'os, des cellules dérivées de tissu mou, des cellules mésothéliales, des cellules stromales spécifiques de tissu, des cellules-souches, des cellules progénitrices, des cellules dérivées d'endoderme, des cellules dérivées d'ectoderme, des cellules dérivées de mésoderme et leurs associations.

8. Tissu ou organe selon l'une quelconque des revendications précédentes, des cellules autres que des cellules musculaires ayant été distribuées sur au moins une surface de la pluralité de couches.

9. Tissu ou organe selon la revendication 8, des cellules autres que des cellules musculaires ayant été bio-imprimées sur au moins une surface de la pluralité de couches.

10. Tissu ou organe selon la revendication 9, les cellules autres que des cellules musculaires ayant été distribuées sur la pluralité de couches sensiblement au même moment où la pluralité de couches a été fabriquée, après fabrication de la pluralité de couches, pendant la maturation de la pluralité de couches ou après maturation de la pluralité de couches.

11. Tissu ou organe selon la revendication 1, le tissu ou l'organe étant une feuille ou un timbre comprenant des cellules musculaires approprié pour la réparation de plaies, le remplacement de tissus ou l'augmentation tissulaire.

12. Procédé de fabrication d'un tissu ou d'un organe implantable artificiel tridimensionnel comprenant une pluralité de couches, le procédé comprenant les étapes consistant à :
a. bio-imprimer au moins un composant par extrusion d'une bio-encre qui est une composition solide ou semi-solide comprenant des cellules musculaires sous une forme sur une surface biocompatible ; et
b. fusionner la bio-encre en une structure cellulaire cohésive ;
le tissu ou l'organe implantable étant pratiquement exempt de tout échafaudage préformé au moment de la bio-impression et au moment de l'utilisation, et à condition que le tissu ou l'organe soit implantable chez un sujet vertébré, soit une feuille et ne soit pas un tube vasculaire, et que la pluralité des couches soit cohérente entre elles.

13. Procédé selon la revendication 12, la bio-encre comprenant en outre des cellules choisies parmi : des cellules endothéliales, des cellules nerveuses, des péricytes, des fibroblastes, des cellules épithéliales spécifiques de tissu, des chondrocytes, des cellules musculaires squelettiques, des cardiomyocytes, des cellules dérivées d'os, des cellules dérivées de tissu mou, des cellules mésothéliales, des cellules stromales spécifiques de tissu, des cellules-souches, des cellules progénitrices, des cellules dérivées d'endoderme, des cellules dérivées d'ectoderme, des cellules dérivées de mésoderme et leurs associations.

14. Procédé selon la revendication 12, comprenant en outre l'étape consistant à bio-imprimer, pulvériser, peindre, appliquer, revêtir par trempage, greffer, ensemencer, injecter ou stratifier des cellules selon la revendication 13 dans ou sur la forme bio-imprimée selon la revendication 12.
